(19) Europäisches Patentamt
European Patent Office
Office européen des brevets

(11) Publication number: **0 347 158 B1**

(12) **EUROPEAN PATENT SPECIFICATION**

(45) Date of publication of patent specification: **15.03.95**

(51) Int. Cl.$^6$: **C07H 17/08**, A61K 31/70

(21) Application number: **89305945.1**

(22) Date of filing: **13.06.89**

The file contains technical information submitted after the application was filed and not included in this specification

(54) **3-deoxy mycaminosyl tylonolide derivatives.**

(30) Priority: **13.06.88 JP 146410/88**
**18.01.89 JP 9160/89**

(43) Date of publication of application:
**20.12.89 Bulletin 89/51**

(45) Publication of the grant of the patent:
**15.03.95 Bulletin 95/11**

(84) Designated Contracting States:
**AT BE CH DE ES FR GB GR IT LI LU NL SE**

(56) References cited:
**GB-A- 2 058 765**
**GB-A- 2 081 711**

(73) Proprietor: **ZAIDAN HOJIN BISEIBUTSU KAGAKU KENKYU KAI**
**14-23, Kamiosaki 3-chome**
**Shinagawa-ku**
**Tokyo (JP)**

(72) Inventor: **Umezawa, Sumio**
**5-1-1-709, Shinjuku**
**Shinjuku-ku**
**Tokyo (JP)**
Inventor: **Tsuchiya, Tsutomu**
**3-1-17-201, Edahigashi**
**Midori-ku**
**Yokohama-shi**
**Kanagawa (JP)**
Inventor: **Takeuchi, Tomio**
**5-1-11-701A Higashi Gotanda**
**Shinagawa-ku**
**Tokyo (JP)**
Inventor: **Kageyama, Shunji**
**2-35-2-306, Kasuga**
**Tsukuba-shi**
**Ibaragi (JP)**
Inventor: **Sakamoto, Shuichi**
**3-17-7-402, Sake-cho**
**Higashimurayama-shi**
**Tokyo (JP)**

(74) Representative: **Geering, Keith Edwin et al**
**REDDIE & GROSE**
**16 Theobalds Road**
**London WC1X 8PL (GB)**

EP 0 347 158 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to macrolactone compounds which have antibacterial activity and so are useful as medicaments (especially antibiotics) for the prevention or treatment of diseases caused by various bacteria. The compounds of the present invention are 3-deoxy mycaminosyl tylonolide derivatives represented by the following formula, and salts thereof:

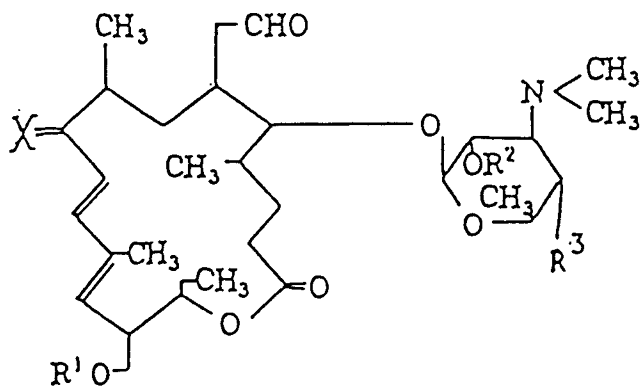

wherein X represents an oxygen atom or =N-OR$^4$ (wherein R$^4$ represents a hydrogen atom or a lower alkyl group); R$^1$ represents a hydrogen atom, an acyl group or an alkylsilyl group; R$^2$ represents a hydrogen atom or an acyl group; and R$^3$ represents a hydrogen atom or a hydroxyl group.

The compounds of the present invention are novel compounds and have chemical-structural characteristics in that the compounds may have acyl group(s) at the 2′-and/or 23-position(s) of 3,4′-dideoxy mycaminosyl tylonolide compounds or of mycaminosyl tylonolide compounds. In the formula (I) compounds "lower" means a chain of 1-5 carbon atoms. Thus "lower alkyl" means a straight or branched chain alkyl having 1 to 5 carbon atoms. Examples of "lower alkyl" are methyl, ethyl, propyl, iso-propyl, butyl, iso-butyl, tert-butyl, pentyl, 1-methylbutyl, 2-methylbutyl, neo-pentyl, etc. The term "alkyl" means a straight or branched chain alkyl having 1 to 10 carbon atoms. Examples of "alkyl" are, in addition to the examples of "lower alkyl", hexyl, 1-methylpentyl, 2-methylpentyl, thexyl, heptyl, 1-methylhexyl, 2-methylhexyl, 2-ethylpentyl,1,3-dimethylpentyl, octyl, nonyl, decyl, etc. Examples of "alkylsilyl" are trimethylsilyl, triethylsilyl, tri-(iso-propyl)silyl, tert-butyldimethylsilyl, tert-butyldiethylsilyl, thexyldimethylsilyl, etc. Herein "thexyl" stands for 2,3,dimethyl-2-butyl

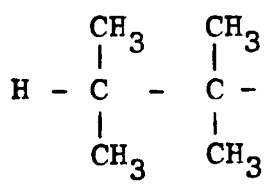

Examples of "acyl" are formyl, acetyl, propionyl, butyryl, iso-butyryl, valeryl, iso-valeryl, pipaloyl, hexanoyl, etc. (namely, alkanoyl groups); acryloyl, methacryloyl, crotonoyl, etc. (namely, lower alkenoyl groups); benzoyl, toluoyl, xyloyl, etc. (namely, aroyl groups); phenylacetyl, phenylpropionyl, phenylhexanoyl, etc. (namely, phenyl alkanoyl groups) The group "N-OR$^4$" has geometrical isomerism; the corresponding compounds (I) include individual such isomers (syn- and anti-isomers) and mixtures thereof.

The present invention also includes salts of compounds of formula (I). Such salts include pharmaceutically acceptable salts, and are, for example, acid addition salts with mineral acids such as hydrochloric acid, sulfuric acid and the like, and with organic acids such as formic acid, toluenesulfonic acid.

The formula (I) compounds may be prepared by various methods. Hereinafter, representative methods are illustrated.

Process variant 1

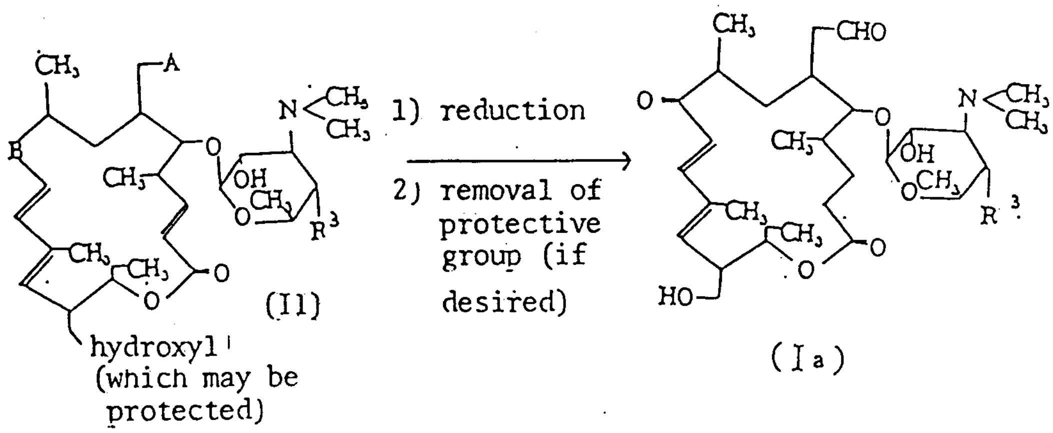


(II)

(Ia)

Process variant 2

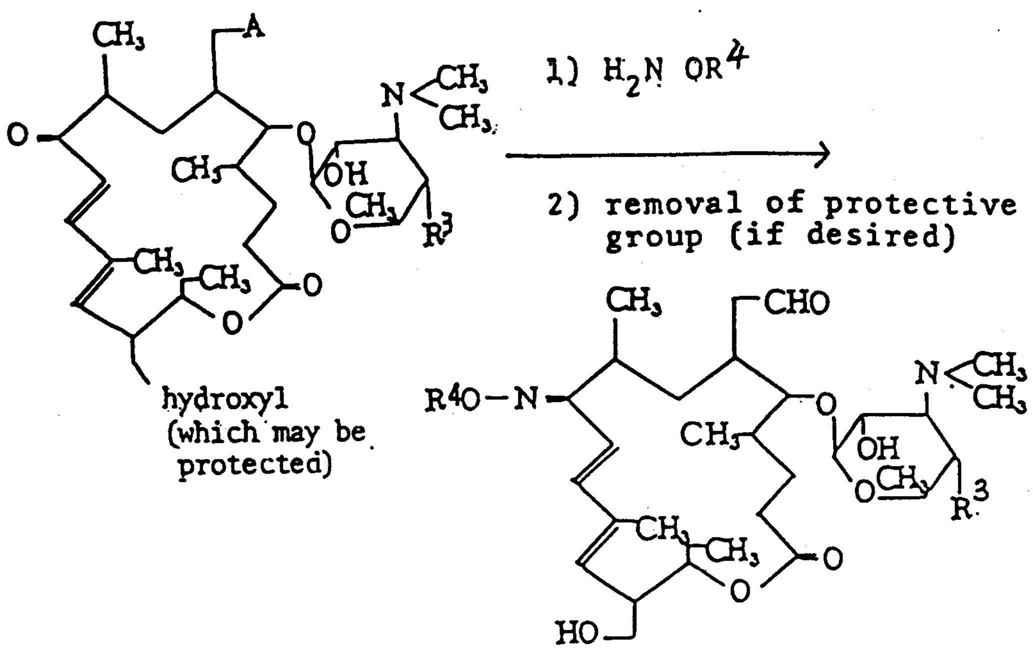


(Ib)

Process variant 3

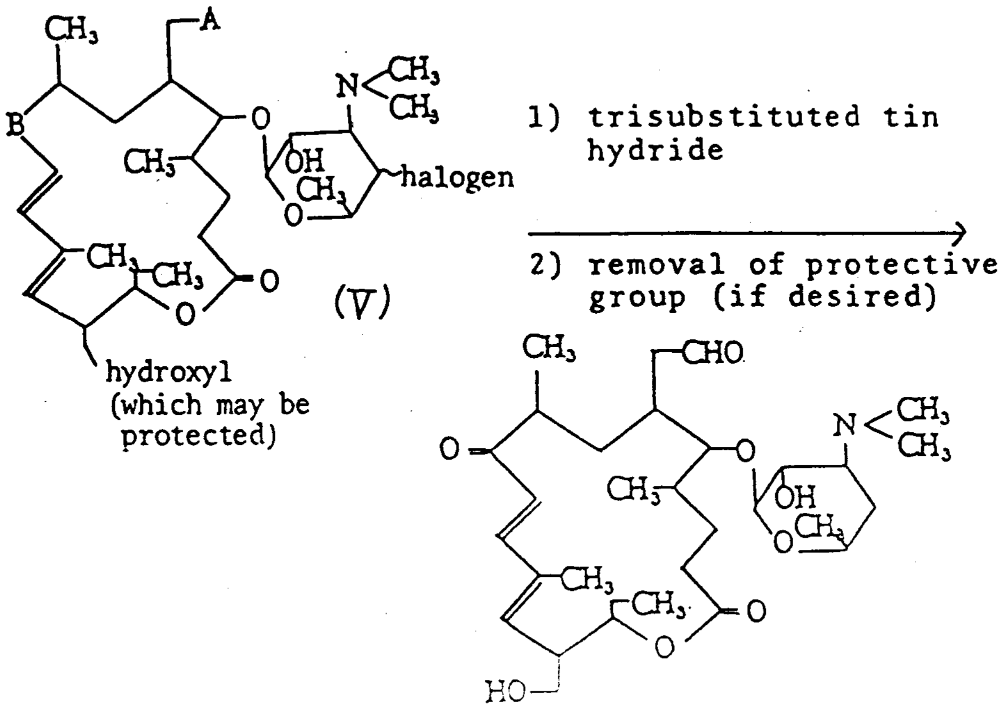


(V)

(Ic)

In the above formula, A represents an aldehyde group which may be protected, and B represents a carbonyl group which may be protected.

Process variant 1:

The formula ($I_a$) compounds (3-deoxy compounds with a 9-carbonyl group) can be prepared by reducing formula (II) compounds (the compounds having a double bond at the 2-position) and optionally removing the protective group if present.

In the formula (II) compounds, as protected-aldehyde or protected-carbonyl, there may be listed acetal (or thioacetal) and ketal (thioketal) types, and examples of these are dimethylacetal (dimetylketal), diethylacetal (diethylketal), diethylthioacetal (diethylthioketal), ethyleneacetal (ethyleneketal), propyleneacetal (propylene ketal) or those substituted by some group such as methyl.

Examples of hydroxyl protective groups are trimethylsilyl, triethylsilyl, tri(iso-propyl)silyl, tri(tert-butyl)-silyl, tert-butyldimethylsilyl, tert-butyldiethylsilyl, thexyldimethylsilyl (2,3-dimethyl-2-butyldimethylsilyl)etc. (silyl type protective group); acetyl, propionyl, butyryl, iso-butyryl, valeryl, iso-valeryl and the like (alkanoyl type protective group); 2-tetrahydrofuranyl, 2-tetrahydropyranyl, and the like.

The reaction may be carried out in an inert solvent, such as benzene, toluene, ether, tetrahydrofuran, dimethylformamide, dimethylsulfoxyde or the like. The reduction of the formula (II) compound may be, for example, carried out by treating with a reducing agent such as di-iso-butyl aluminum hydride. The reaction temperature may be suitably controlled according to the kind of starting compound and reducing agent, etc., but reaction is preferably under cooling.

When the formed compound has a protective group, this is removed, if desired. The removal can be performed in conventional manner, for example, by treating with mineral acid such as hydrochloric acid, sulfuric acid or the like or with organic acid such as trifluoroacetic acid or the like;
removal of a silyl-type protective group can be performed by treating both tetrabutylammoniumfluoride, hydrochloric acid, acetic acid, etc.

Process variant 2

This method can be performed by reacting amino-compound ($H_2N-OR^4$) with formula (III) compound (mycaminosyl tylonolide whose aldehyde group may be protected, or its 4'-deoxy derivative), and then removing the protective group when the aldehyde- or carbonyl-group is protected.

The reaction of compounds (III) and (IV) can be carried out in an inert solvent; the formula (IV) compound may be in the form of free base or of salt with acid. In the former case the reaction may be carried out after addition of acid such as pyridine-p-toluene-sulfonate, 10-camphorsulfonic acid or the like; in the latter case the reaction may be carried out after addition of inorganic base such as sodium hydrogen carbonate, sodium carbonate, potassium carbonate or the like, or addition of organic base such as pyridine, triethylamine, piperidine or the like. When such acid or base is added, the pH of the reaction solution is preferably controlled to 4-5. The reaction solvents are, for example, water, alcohol, tetrahydrofuran, acetonitrile, benzene and the like; The reaction is preferably performed at room temperature or with heating (by refluxing) for 1-2 hours to 2-3 days.

The removal of protective groups from the formed compounds can be carried out in conventional manner as described in the foregoing Process Variant 1.

Process variant 3

The method is performed by replacing 4′-halogen atom with hydrogen atom, and the reaction can be carried out by reacting reducing agent (in particular, trisubstituted tin hydride) with formula (V) compound.

Examples of trisubstituted tin hydride are triethyl tin hydride, tri-n-butyl tin hydride and the like (trialkyltin hydrides);triphenyl tin hydride (triaryl tin hydrides); and the like. Preferable examples of the reaction solvent are toluene, benzene, dioxane, tetrahydrofuran and the like (namely, aprotic solvent which has no halogen atom and is difficult to reduce). The reaction may be performed at room temperature or under heating. If necessary, in order to initiate the reaction, radical initiator such as alpha, alpha-azobis-iso-butylonitrile may added to the reaction solution.

Protective groups can be removed in conventional manner as described in the foregoing Process variant 1.

Process variant 4

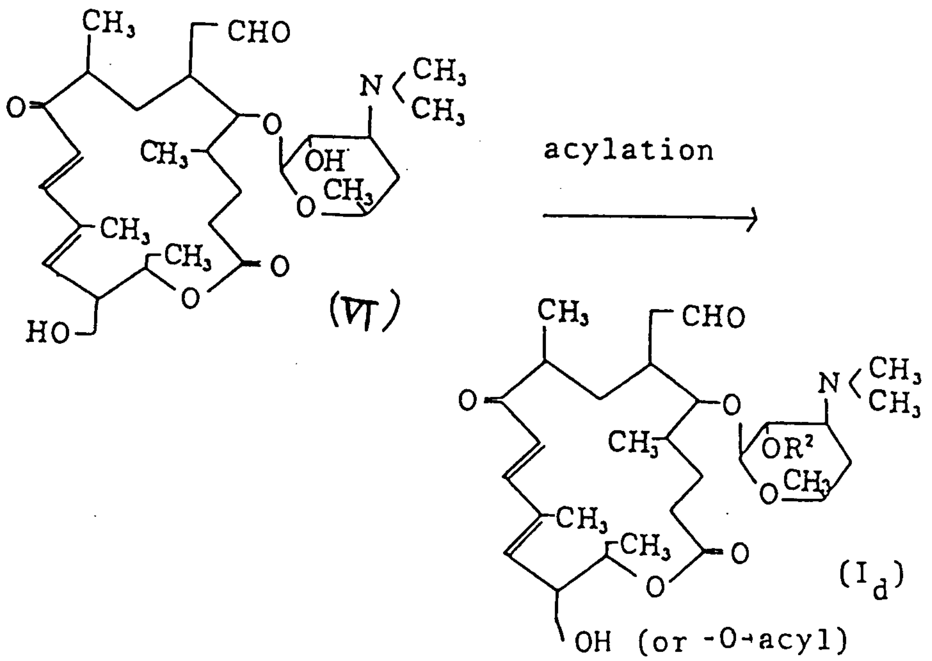


Process variant 5

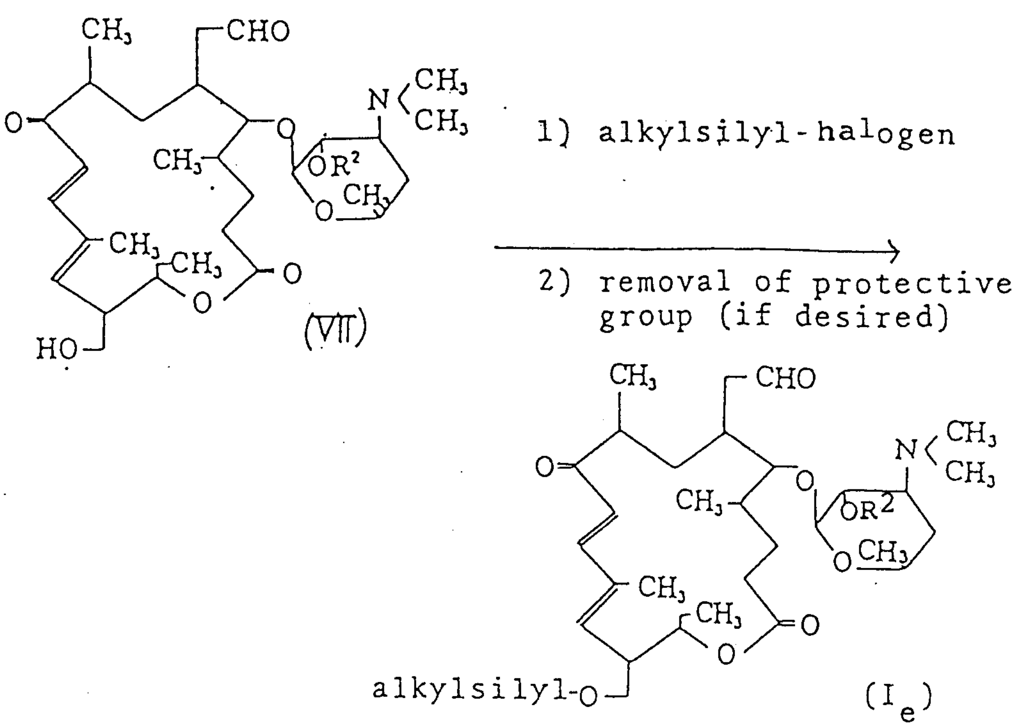


Process variant 4

Formula ($I_d$) compounds of the present invention can be prepared by acylating formula (VI) compounds (3,4'-dideoxy mycaminosyl tylonolide derivatives).

The acylating reaction may be performed using various acylating agents such as acid halogenide (for example, acetyl chloride, acetyl bromide, propionyl chloride, pivaloyl chloride, benzoyl chloride), acid anhydride (e. g. acetic acid, anhydride, benzoic acid, anhydride), active ester (acid p-nitrophenyl ester, acid 2,4-dinitrophenyl ester, etc.) under suitable reaction condition The acid may be used in the form of free acid or its salt, and in this case, dehydrating agent such as hydrochloric acid or sulfuric acid, etc. is used in the reaction.

Examples of the reaction solvent are water, acetone, dioxane, acetonitrile, chloroform, benzene, dichloromethane, tetrahydrofuran, ethyl acetate, N,N-dimethylformamide, pyridine (that is, any solvent which does not take part in the reaction); the solvents may be used as a mixture thereof. The reaction is preferably conducted under cooling or under heating.

The starting compound (VI) can be prepared by a method described in unexamined Japanese patent publication Sho. 63-146410.

Process variant 5

Formula ($I_e$) compounds can be prepared by reacting formula (VII) compounds with alkylsilyl halide (alkylsilyl-halogen), and then, if desired, removing the 2′-acyl group.

Examples of alkylsilyl halide are trimethylsilyl chloride, triethylsilyl bromide, tertbutyldimethylsilyl chloride, thexyldimethylsilyl chloride. The reaction is preferably performed in the presence of inorganic or organic base such as sodium carbonate, potassium carbonate, sodium hydrogen carbonate, pyridine, lutidine, picoline, imidazole.

Examples of the reaction solvent are acetone, acetnitrile, tetrahydrofuran, benzene, chloroform, dimethylformamide.

The removal of acyl groups from the formed compound can be performed using inorganic acid such as hydrochloric acid, sulfuric acid, etc. or organic acid such as trifluoroacetic acid, etc., or by allowing the formed compound to stand as it is, in alcohols such as methanol, ethanol, etc.

The formula (I) compound thus prepared may be separated and purified by conventional methods such as extraction, recrystallization, column chromatography, etc.

The compounds of this invention have shown antibacterial activity against various pathogens including gram-positive and -negative bacteria. Thus, the compounds are useful as medicaments (especially antibiotics) for the prevention or treatment of diseases caused by such bacteria.

Concerning the antimicrobial activity of the compounds of this invention, the following experiment has been done.

Staph. aureus Smith (bacteria amount: $10^6$ CFU/mouse) were injected intraperitoneally into 6 healthy mice (one group), and after 2 hours each Sample was given orally, and the survival number after one week observed. The compounds of the present invention have shown excellent in vivo preventing and treating effect ($ED_{50}$: 50-150 mg/kg).

GB-A-2081711 discloses tylosin derivatives which differ from those of the present invention in that they are not 3-deoxy but have a 3-hydroxy or -alkanoyloxy group, or are 3-deoxy but have a double bond at the 2-position (as in formula (II) above).

Medicaments containing as active component the com pounds of the present invention may be prepared using pharmaceutical carriers and excipients as conventional. The types of administration may include oral administration of tablets, granules, powders, capsules and the like, and parenteral administration by injection and the like. The dosage may be sutably determined depending upon the conditions, but, for an adult, the total dosage is e.g. 50 - 2,000 mg per day - preferably administered in one to four doses per day.

Examples

The following Examples further detail the preparative methods of the compounds of this invention. Some of the starting compounds are novel, so their manufacturing methods are also shown in the following Reference Examples. In the following Examples, "Mass" means mass spectrum, and $^1$H-nmr means hydrogen nuclear magnetic resonance spectrum.

Reference Example 1

23-O-thexyldimethylsilyl-mycaminosyl tylonolide 9,20-bis(ethyleneacetal)

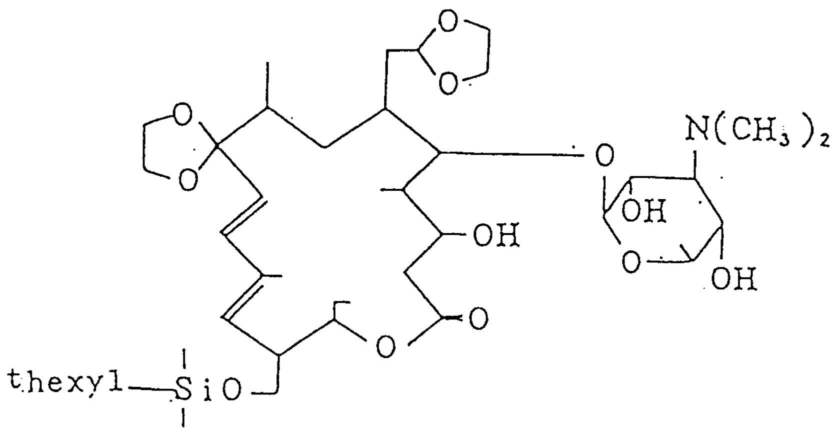

1.00 g of mycaminosyl tylonolide 9,20-bis(ethyleneacetal) was dissolved in 8 ml of dry dimethylformamide, and after adding 200 mg of imidazole and 0.39 g of thexyldimethylchlorosilane

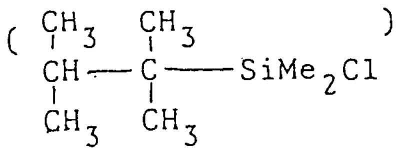,

the reaction mixture was kept at room temperature for 6 hours. The reaction mixture was concentrated, and to the residue was added saturated aqueous sodium hydrogencarbonate (100 ml). The mixture thus formed was extracted with chloroform. The organic layer separated was washed with water, and dried to give a syrupy which was purified by chromatography (Wako gel C-200: 80 g; chloroform:methanol:conc.aqueous ammonia = 15:1:0.1) to give 23-O-thexyldimethylsilyl mycaminosyl tylonolide 9,20-bis(ethyleneacetal) as solid material (1.12 g; yield 93 %).

Physico-chemical character:

(i) $[\alpha]_D^{23}$ : -9° (c1, $CHCl_3$)
(ii)

| Elemental analysis ($C_{43}H_{77}NO_{12}Si$) | | | |
|---|---|---|---|
| | C | H | N |
| Found (%) | 61.95 | 9.20 | 1.68 |
| Calcd.(%) | 62.36 | 9.37 | 1.69 |

(iii) Mass m/z = 828 ($M^+$)

Reference Example 2

2',4'-di-O-acetyl-23-O-thexyldimethylsilyl mycaminosyl tylonolide-9,20-bis(ethyleneacetal)

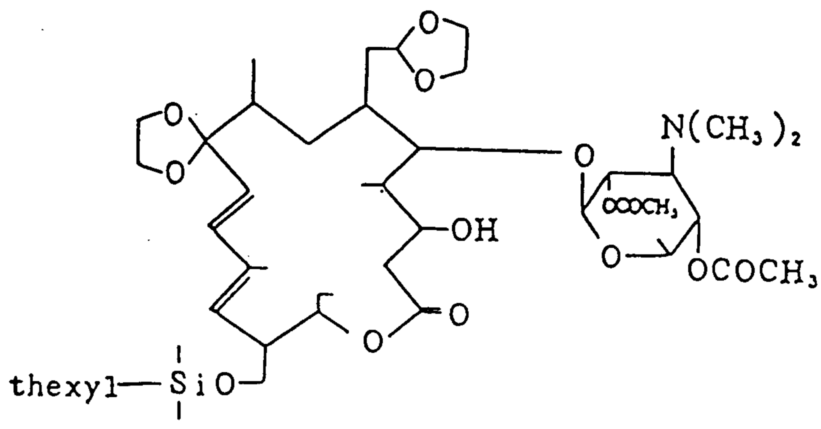

2.20 g of 23-O-thexyldimethylsilyl mycaminosyl tylonolide 9,20-bis(ethyleneacetal) was dissolved in 22 ml of dry acetonitrile, and after adding 0.58 ml of acetic anhydride, the mixture was kept at room temperature overnight. The reaction mixture was concentrated, and after adding saturated aqueous sodium hydrogencarbonate (100 ml), the mixture was extracted with toluene, and stirred. The organic layer was washed with water, dried, and concentrated to give solid material, which was purified by chromatography [silica gel] (Wako gel C-200: 200 g; cylcohexane:acetone =7:2) to give colorless solid 2',4'-di-O-acetyl-23-O-thexyldimethylsilyl mycaminosyl tylonolide 9,20-bis(ethyleneacetal) [2.04 g, yield: 84 %).

Physico-chemical character:

(i) $[\alpha]_D^{25}$ : -40° (c1, $CHCl_3$)
(ii)

| Elemental analysis ($C_{47}H_{81}NO_{14}Si$) | | | |
|---|---|---|---|
| | C | H | N |
| Found.(%) | 61.79 | 8.96 | 1.48 |
| Calcd.(%) | 61.88 | 8.95 | 1.54 |

(iii) Mass m/z = 912($M^+$)
(iv) $^1H$-nmr ( $CDCl_3$ TMS internal standard)
δ 2.00, 2.04 (each 3H, s, $CH_3$ of 2',4'-$COCH_3$)

Reference Example 3

2',4'-di-O-acetyl-3-O-mesyl-23-O-thexyldimethylsilyl mycaminosyl tylonolide-9,20-bis(ethyleneacetal)

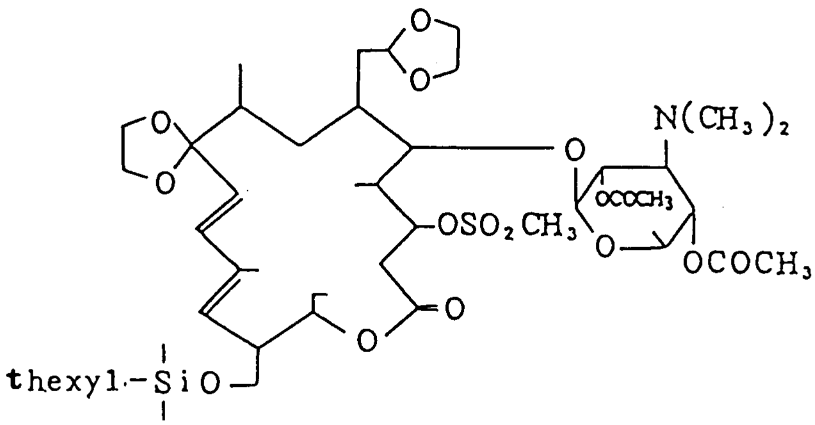

700 mg (0.77 mmol) of 2',4'-di-O-acetyl-23-O-thexyldimethylsilyl mycaminosyl tylonolide-9,20-bis-(ethyleneacetal) was dissolved in 2 ml of dry pyridine, and after adding 0.18 ml (2.3 mmol) of methanesulfonyl chloride, the mixture was kept at room temperature for 3 hours. The reaction solution was poured into 100 ml of saturated aqueous sodium hydrogen carbonate, and extracted with 50 ml of toluene 3 times. The organic layer was washed with water, dried, and concentrated to give pale yellow syrup, which was purified by silica gel column chromatography (Wako gel C-200: 35 g; cyclohexane:acetone = 3:1) to give 723 mg (yield: 95 %) of colorless solid 2',4'-di-O-acetyl-3-O-mesyl-23-O-thexyldimethylsilyl mycaminosyl tylonolide 9,20-bis(ethyleneacetal).

Physico-chemical character:

(i) $[\alpha]_D^{25}$ : -51° (c1, $CHCl_3$)
(ii)

| Elemental analysis ($C_{48}H_{83}NO_{16}SSi$) | | | |
|---|---|---|---|
| | C | H | N |
| Found (%) | 58.04 | 8.62 | 1.36 |
| Calcd.(%) | 58.22 | 8.45 | 1.41 |

(iii) Mass m/z = 990($M^+$)
(iv) $^1$H-nmr ($CDCl_3$, TMS)
δ; 2.02, 2.04 (each 3H, s, $CH_3$ of 2',4'-$COCH_3$) 3.15 (3H, s, $CH_3$ of $3OSO_2CH_3$)

Reference Example 4

2-dehydro-2-en-3-deoxy-23-O-thexyldimethylsilylmycaminosyl tylonolide 9,20-bis(ethyleneacetal)

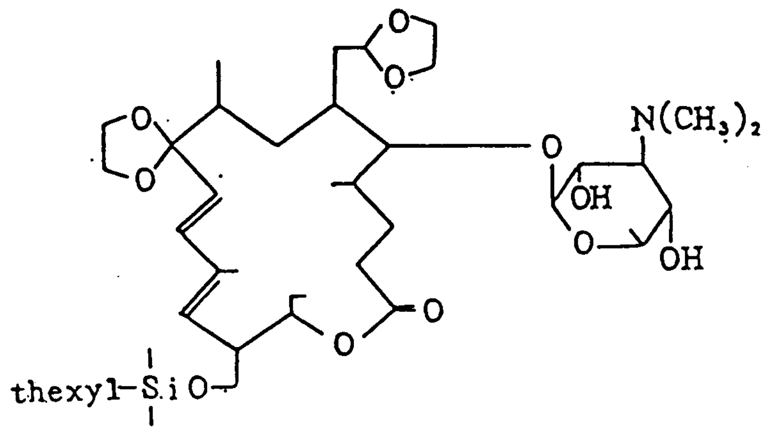

51 mg of 2′,4′-di-O-acetyl-23-O-thexyldimethylsilylmycaminosyl tylonolide 9,20-bis(ethyleneacetal) was dissolved in 1.5 ml of methanol, and after adding 0.5 ml of conc. aqueous ammonia, the mixture was kept at room temperature for 3 hours. The reaction solution was concentrated, and the residue was extracted with chloroform. The chloroform layer was concentrated; and the solid material obtained was dissolved in 1 ml of methanol, and kept at 50 °C overnight. The reaction solution was neutralized using saturated aqueous sodium hydrogen carbonate, and extracted with chloroform. The organic layer was washed with saturated aqueous NaCl, dried, and concentrated to give pale yellow syrup, which was purified by silica gel column chromatography (Wako gel C-200: 5 g; chloroform:methanol:conc.aqueus ammonia = 15:1:0.1) to give 39,9 g (yield: 96 %) of colorless solid 2-dehydro-2-en-3-deoxy-23-O-thexyldimethylsilylmycaminosyl tylonolide 9,20-bis(ethyleneacetal).

Physico-chemical character:

(i) $[\alpha]_D^{25}$ : -34 °C (c1, $CHCl_3$)
(ii)

| Elemental analysis ($C_{43}H_{75}NO_{11}Si$) | | | |
|---|---|---|---|
| | C | H | N |
| Found (%) | 63.47 | 9.36 | 1.68 |
| Calcd.(%) | 63.75 | 9.33 | 1.73 |

(iii) Mass m/z = 810($M^+$)

Reference Example 5

(1) 4′-O-benzylsulfonyl-3-deoxy-23-O-thexyldimethylsilylmycaminosyl tylonolide 9,20-bis(ethyleneacetal)

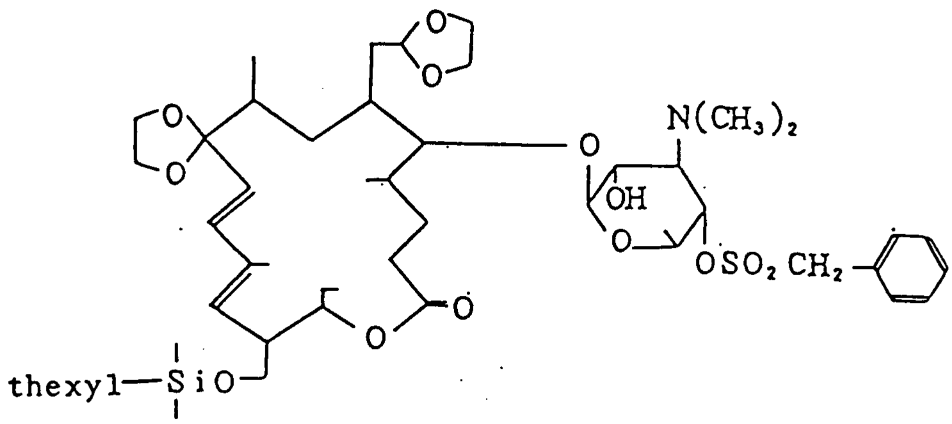

1.30 g of 3-deoxy-23-O-thexyldimethylsilyl mycaminosyltylonolide 9,20-bis(ethyleneacetal) was dissolved in 26 ml of dry pyridine, and was cooled to -40 ° C. After adding 459 mg of benzylsulfonyl chloride, the mixture was allowed to react for 3 hours. After adding 0.5 ml of water, the reaction mixture was allowed to come to room temperature. After stirring for 1 hour, the mixture was concentrated. After adding 100 ml of saturated aqueous sodium hydrogen carbonate, the mixture was extracted with chloroform. The organic layer was washed with saturated aqueous sodium chloride, dried over magnesium sulfate, and concentrated to give unstable pale yellow solid material (1.56 g).

(2) 3,4′-dideoxy-4′-iodo-23-O-thexyldimethylsilylmycaminosyl tylonolide 9,20-bis(ethyleneacetal)

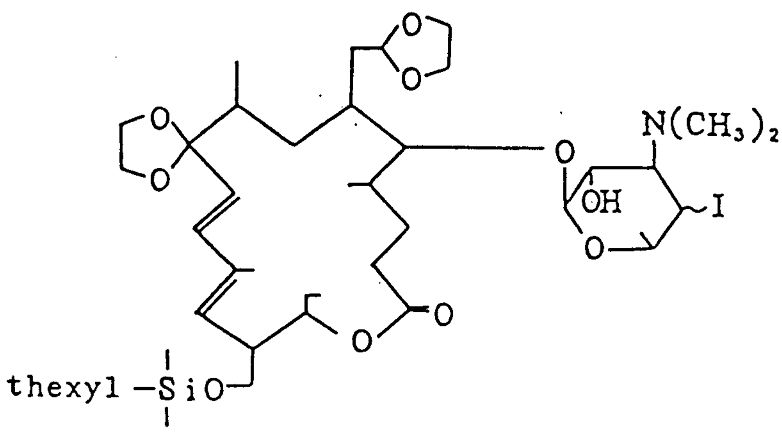

4′-O-benzylsulfonyl-3-deoxy-23-O-thexyldimethylsilylmycaminosyl tylonolide 9,20-bis(ethyleneacetal) [crude product, 1.56 g obtained at (1) above] was dissolved in 24 ml of dry 2-butanone, and after adding 366 mg of sodium iodide, the mixture was stirred under heating under nitrogen gas atmosphere at 80 ° C. After 30 minutes, the reaction solution was filtered, and washed. The filtered solution and washing solution were combined, and concentrated. The residue was extracted with ethyl acetate. The organic layer was washed with saturated aqueous sodium hydrogen carbonate, 0.1 M aqueous sodium thiosulfate and saturated aqueous sodium chloride, successively, dried over magnesium sulfate, and concentrated to give yellow syrup.

This was purified by silica gel column chromatography (Wako gel C-200; 80 g; cyclohexane: acetone = 7:2) to give 3,4'-dideoxy-4'-iodo-23-O-thexyldimethylsilyl mycaminosyl tylonolide 9,20-bis(ethyleneacetal) as colorless solid material (1.04 g, yield from (1) above: 71 %).

Physico-chemical character:

(i)

$$[\alpha]_D^{20.5}:$$

-73° (c1, $CHCl_3$)

(ii)

| Elemental analysis ($C_{43}H_{76}NO_{10}SiI$) | | | | |
|---|---|---|---|---|
| | C | H | N | I |
| Found (%)<br>Calcd.(%) | 56.39<br>56.01 | 8.24<br>8.31 | 1.46<br>1.52 | 14.14<br>13.76 |

(iii) Mass (FAB) m/z = 922($M^+$)

Reference Example 6

3,4'-dideoxymycaminosyl tylonolide dimethylacetal 300 mg of 3,4′-dideoxymycaminosyl tylonolide was dissolved in 5.0 ml of dry methanol, and after adding 150 mg of p-toluenesulfonic acid, the mixture was allowed to react at room temperature for 1 hour. After adding 0.12 ml of triethylamine, the reaction solution was concentrated. To the residue was added 15 ml of saturated aqueous sodium hydrogen carbonate, and the mixture was extracted with chloroform. The extract solution was washed, dried over magnesium sulfate, and concentrated to give foamy solid material. This material was purified by silica gel column chromatography (Wako gel:C-200: 6 g; chloroform:methanol:conc. aqueous ammonia = 10:1:0.1) to give 306 mg of 3,4′-dideoxymycaminosyltylonolide dimethylacetal (yield: 94 %).

Physico-chemical character:

(i) $[\alpha]_D^{24}$ : +8° (c2, $CHCl_3$)

(ii)

| Elemental analysis | | | |
|---|---|---|---|
| | C | H | N |
| Found (%)<br>Calcd.(%) | 63.95<br>63.84 | 9.26<br>9.42 | 2.20<br>2.25 |

(iii) Mass m/z = 611($M^+$)

Example 1

(1) 3-Deoxy-23-O-thexyldimethylsilylmycaminosyltylonolide 9,20-bis(ethyleneacetal)

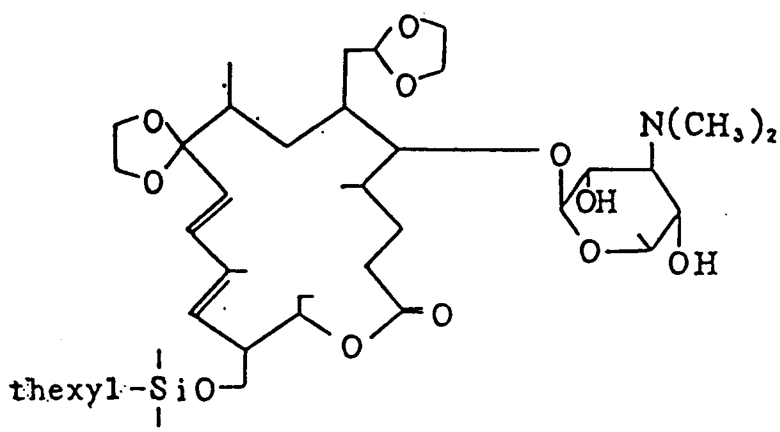

2.00 g (2.47 mmol) of 2-dehydro-2-en-3-deoxy-23-O-thexyldimethylsilylmycaminosyl tylonolide 9,20-bis-(ethyleneacetal) was dissolved in dry toluene, and the mixture was cooled to -60 °C, and 7.4 ml of 1.5 M diisobutylaluminum hydride-toluene solution was poured into the above mixture under nitrogen gas stream with stirring. After 30 minutes, 3 g of sodium sulfate $10H_2O$ was added to the mixture, the reaction was stopped, and after adding 20 ml of water containing 1.33 ml of acetic acid, the mixture was allowed to come to room temperature. After adding saturated aqueous sodium hydrogen carbonate and stirring, the mixture was extracted with chloroform. The organic layer was washed with water, dried and concentrated to give colorless foamy solid material (1.95 g). This was purified by silica gel column chromatography (Wako gel C-200: 200 g; chloroform:methanol: conc. aqueous ammonia = 18:1:0.1) to give 1.09 g of 3-deoxy-23-O-thexyldimethylsilyl mycaminosyltylonolide 9,20-bis(ethyleneacetal) (yield: 55%) as colorless solid material.

Physico-chemical character:

(i) $[\alpha]_D^{21}$ : -52 ° (c1, $CHCl_3$)
(ii)

| Elemental analysis ($C_{43}H_{77}NO_{11}Si$) | | | |
|---|---|---|---|
| | C | H | N |
| Found (%) | 63.08 | 9.49 | 1.73 |
| Calcd.(%) | 63.59 | 9.56 | 1.72 |

(2) 3-Deoxy-mycaminosyltylonolide

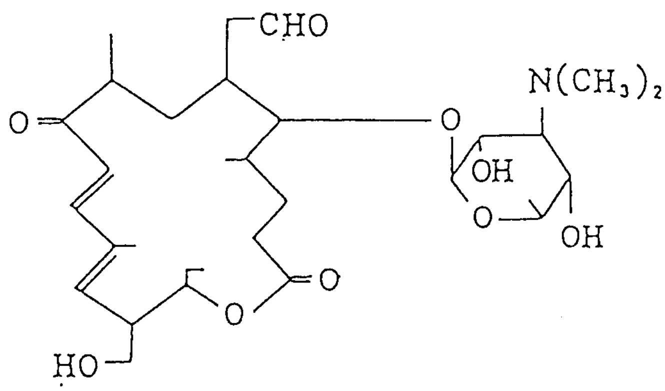

0.60 g of 3-deoxy-23-O-thexyldimethylsilyl mycaminosyltylonolide 9,20-bis(ethyleneacetal) was dissolved in 9 ml of tetrahydrofuran, and after adding 1.13 ml of 1 M tetrabutylammonium fluoride-tetrahydrofuran solution, the mixture was allowed to stand at room temperature. After confirming termination of the de-silylation, the reaction solution was concentrated (1/5); after adding 50 ml of saturated aqueous sodium hydrogen carbonate, the mixture was extracted with choroform. The solution was washed with water, dried and concentrated to give crude product of 3-deoxy-maycaminosyltylonolide-9,20-bis(ethyleneacetal). This product was dissolved in 6 ml of acetonitrile, and after adding 24 ml of 0.1 M aqueous hydrochloric acid, the formed white solution was stirred. After 6 hours and then adding 60 ml of saturated aqueous sodium hydrogen carbonate, the mixture was extracted with chloroform. The organic layer was concentrated to give pale yellow solid material. This was purified by silica gel colum chromatography (Wako gel C-200: 40 g; chloroform:methanol:conc. aqueous ammonia = 15:1:0.1) to give 383 mg of colorless solid 3-deoxymycaminosyl tylonolide (yield: 89 % from the product of Example 1 (1)).

Physico-chemical character:

(i) $[\alpha]_D^{22}$ : -23 ° C (c1, $CHCl_3$)
(ii)

| Elemental analysis ($C_{31}H_{51}NO_9$: $H_2O$) | | | |
|---|---|---|---|
| | C | H | N |
| Found (%) | 62.29 | 8.80 | 2.29 |
| Calcd.(%) | 62.08 | 8.73 | 2.33 |

(iii) Mass m/z 582($M^+$)
(iV) $^1$H-nmr ($CDCl_3$, TMS)
δ 0.94 (3H, t, 17-$CH_3$), 0.98 (3H, d, 18-$CH_3$), 1.22 (3H, d, 21-$CH_3$), 1.25 (3H, d, 6′-$CH_3$), 1.85 (3H, d, 22-$CH_3$), 2.35 (1H, t, H-3′), 2.49 (6H, s, 3′-N ($CH_3$)$_2$), 3.02 (1H, t, H-4′), 3.23 (1H, dd, H-5′), 3.48 (1H, dd, H-2′), 4.23 (1H, d, H-1′), 4.88 (1H, m, H-15), 5.82 (1H, bd, H-13; $J_{13,14}$ = 10Hz), 6.34 (1H, d, H-10; $J_{10,11}$ = 16Hz), 7.30 (1H, d, H-11) 9.68 (1H, s, H-20)

Example 2

(1) 3,4'-Dideoxy-23-O-thexyldimethylsilyl-mycaminosyltylonolide 9,20-bis(ethyleneacetal)

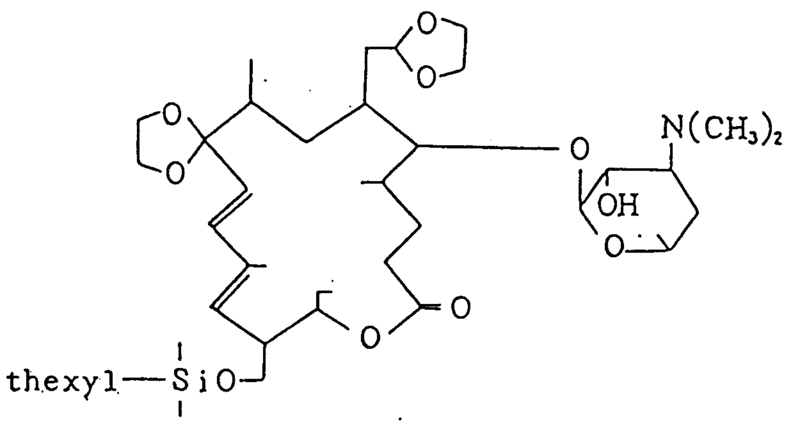

1.04 g of 3,4'-dideoxy-4'-iodo-23-O-thexyldimethylsilyl mycaminosyltylonolide 9,20bis(ethyleneacetal) was dissolved in 20 ml of dry benzene, and after adding 0.91 ml (3.4 mmol) of tributyltin hydride and 37 mg of azodiisobutyronitrile, the mixture was allowed to react for 2 hours at 80 °C. Then the reaction solution was concentrated, and the residue was purified by silica gel column chromatography (Wako gel C-200: 50 g; cyclohexane:acetone = 3:1 → chloroform (600 ml) → chloroform: methanol:conc. aqueous ammonia = 10:1:0.1) to give 0.79 of colorless solid 3,4'-dideoxy-23-O-thexyldimethylsilylmycaminosyltylonolide 9,20-bis(ethyleneacetal). [yield: 88 %]

Physico-chemical character:

(i) $[\alpha]_D^{20}$ : -38° (c1, $CHCl_3$)
(ii) Mass m/z = 795($M^+$)

(2) 3,4'-Dideoxy-mycaminosyltylonolide

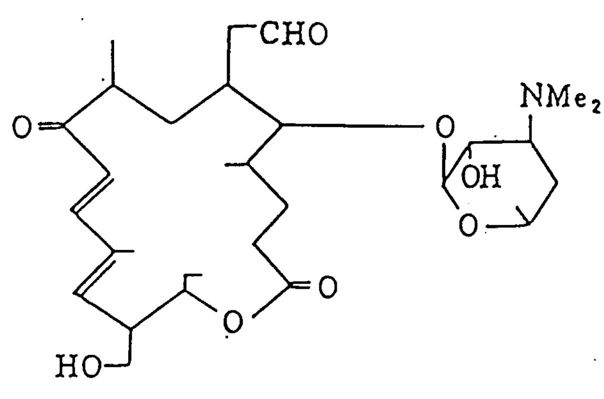

0.73 g of 3,4'-dideoxy-23-O-theyxldimethylsilyl mycaminosyltylonolide 9,20-bis(ethyleneacetal) was dissolved in 12 ml of tetrahydrofuran, and after adding 1.65 ml of 1 M tetrabutylammonium fluroride-tetrahydrofuran solution, the mixture was allowed to react for 5 hours. The reaction mixture was concentrated, and the residue was extracted with chloroform and washed with saturated aqueous sodium hydrogen carbonate and saturated aqueous sodium chloride. The organic layer was dried over magnesium sulfate, and concentrated to give pale yellow solid material (0.72 g) of the crude product of 3,4'-dideoxy mycaminosyltylonolide 9,20-bis(ethyleneacetal). This product was dissolved in 8 ml of acetonitrile, and after

adding 24 ml of 0.1 M aqueous hydrochloric acid, the mixture was allowed to react at 37°C overnight. To the reaction solution was added 70 ml of saturated aqueous sodium hydrogen carbonate, and the mixture was extracted with chloroform. The extract solution was washed with saturated aqueous sodium hydrogen carbonate, dried over magnesium sulfate, and concentrated to give pale yellow syrup This was purified by silica gel column chromatography (Wako gel C-200; 35 g; chloroform: MeOH:conc. aqueous ammonia = 12:1:0.1) to give 3,4′-dideoxymycaminosyltylonolide (511 mg, colorless material) [yield: 99 %].

Physico-chemical character:

(i) $[\alpha]_D^{19}$ : -21° (c1, $CHCl_3$)
(ii)

| Elemental analysis ($C_{31}H_{51}NO_8.H_2O$) | | | |
|---|---|---|---|
| | C | H | N |
| Found (%) | 63.81 | 8.80 | 2.50 |
| Calcd.(%) | 63.78 | 8.97 | 2.40 |

(iii) Mass m/z = 566($M^+$)
(iv) $^1H$-nmr ($CDCl_3$, TMS)
δ 0.94 (3H, t, 17-$CH_3$), 1.04 (3H, d, 18-$CH_3$), 1.85 (3H, d, 22-$CH_3$), 2.26 (6H, s, 3′-$N(CH_3)_2$), ~2.90 (1H, m, H-14), 3.19 (1H, dd, H-2′), 4.19 (1H, d, H-1′), 4.88 (1H, m, H-15), 5.83 (1H, d, H-13; $J_{13,14}$ = 10Hz), 6.35 (1H, d, H-10; $J_{10,11}$ = 16 Hz), 7.30 (1H, d, H-11) 9.70 (1H, s, H-20)

Example 3

(1) 9-Deoxo-3,4′-dideoxy-9-N-methoxyimino-mycaminosyltylonolide dimethylacetal

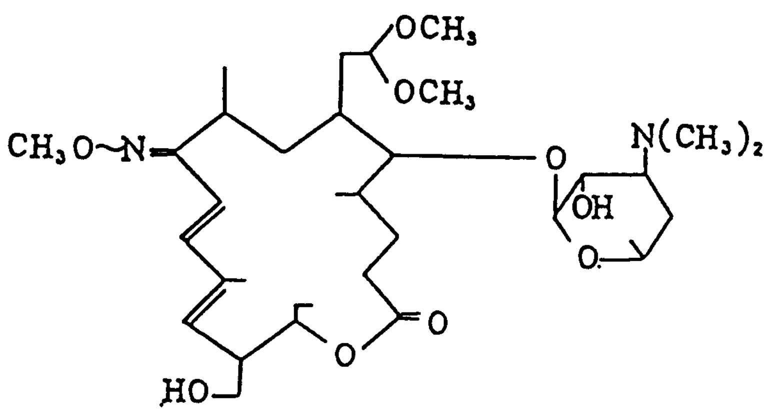

293 mg of 3,4′-dideoxy mycaminosyltylonolide dimethylacetal (0.48 mmol) was dissolved in dry methanol, and after adding 85 µl (0.98 mmol) of dry pyridine and 81 mg (0.97 mmol) of methoxyamine hydrochloride, the mixture was allowed to react overnight. The reaction solution was concentrated, and after adding to the residue 15 ml of saturated aqueous sodium hydrogen carbonate, the mixture was extracted with chloroform, and the organic layer was washed with saturated aqueous sodium chloride, dried and concentrated to give foamy solid material (280 mg). This product is a mixture of two kinds of 9-N-methoxyimino compound (5:1). This mixture was purified by silica gel column chromatography (Wako gel C-200: 50 g; chloroform:methanol:conc. aqueous ammonia = 15:1:0.1) to give colorless solid material of the main product of 9-deoxo-3,4′-dideoxy-9-N-methoxyimino-mycaminosyltylonolidedimethylacetal (163 mg).

Physico-chemical character:

(i) $[\alpha]_D^{22}$ : -30° (c1, $CHCl_3$)
(ii)

| Elemental analysis ($C_{34}H_{60}N_2O_9$:0.5$H_2O$) | | | |
|---|---|---|---|
| | C | H | N |
| Found (%) | 62.85 | 9.34 | 4.24 |
| Calcd.(%) | 62.83 | 9.46 | 4.31 |

(iii) Mass m/z = 609 ($M^+$-$CH_3O$)

(2) 9-Deoxo-3,4′-dideoxy-9-N-methoxyiminomycaminosyltylonolide

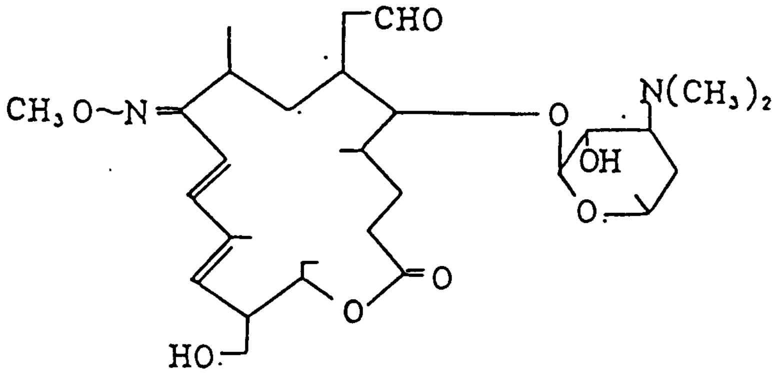

246 mg of 9-deoxo-3,4′-dideoxy-9-N-methoxyiminomycaminosyltylonolide dimethylacetal (two kinds of 9-N-methoxyimino compound, 5:1 mixture) was dissolved in 2.5 ml of acetonitrile, and after adding 10 ml of 0.1 M aqueous hydrochloric acid, the mixture was stirred for 4 hours at room temperature. The reaction solution was concentrated (1/3), and after adding 25 ml of saturated aqueous sodium hydrogen carbonate, the mixture was extracted with chloroform. The extract solution was washed with saturated aqueous sodium chloride, dried and concentrated, and the solid material obtained was purified by silica gel column chromatography (Wako gel C-200: 30g; chloroform:methanol:conc. aqueous ammonia = 15:1:0.1) to give colorless solid (205 mg) 9-deoxo-3,4'-dideoxy-9-N-methoxyimino-mycaminosyltylonolide (yield: 90 %). This material is a mixture of two kinds of 9-N-methoxyimino compound (5:1).

Physico-chemical character:

(i) $[\alpha]_D^{22}$ : -59° (c1, $CHCl_3$)
In similar fashion, 17 mg of the main product of 9-deoxo-3,4'-dideoxy-9-N-methoxyimino-mycaminosyltylonolide dimethylacetal was allowed to react and treated to give the main product of 9-deoxo-3,4'-dideoxy-9-N-methoxyimino-mycaminosyltylonolide.

Physico-chemical character:

(i) $[\alpha]_D^{22}$ : -70° (c1, $CHCl_3$)
(ii) Mass m/z = 595($M^+$)

Example 4

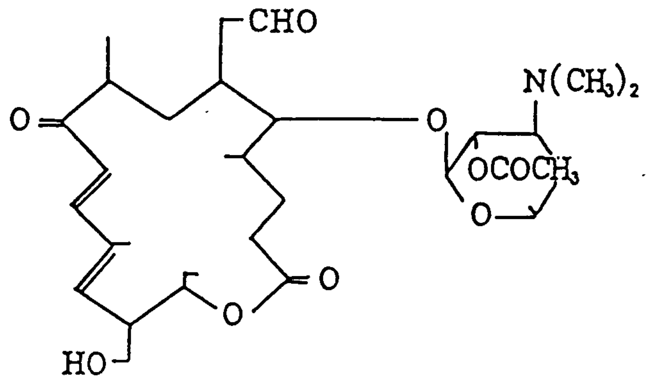

3.00 g of 3,4'-dideoxymycaminosyltylonolide was dissolved in 30 ml of dry acetonitrile, and after adding 0.65 ml of acetic anhydride, the mixture was allowed to react at room temperature for 3 hours. The reaction solution was concentrated, and after adding 100 ml of saturated aqueous sodium hydrogen carbonate, the mixture was extracted with ethyl acetate. The extract solution was washed with water, dried and concentrated to give pale yellow solid material. This was purified by silica gel column chromatography (Wako gel C-200: 150 g; chloroform: methanol = 10:1) to give 2.95 g (yield: 92 %) of colorless solid 2'-O-acetyl-3,4'-dideoxymycaminosyl tylonolide.

Physico-chemical character:

$[\alpha]_D^{26}$ : -1 ° (c1, $CHCl_3$)
(ii)

| Elemental analysis ($C_{33}H_{53}NO_9.H_2O$) | | | |
|---|---|---|---|
| | C | H | N |
| Found(%) | 63.40 | 8.47 | 2.12 |
| Calcd.(%) | 63.33 | 8.86 | 2.24 |

(iii) Mass m/z = 608($M^+$ + 1)
(iv) $^1$H-nmr ($CDCl_3$, TMS internal standard)
$\delta$ 2.06 (3H, s, 2'-$OCOCH_3$ :$CH_3$) 2.26 (6H, s, 3'-$N(CH_3)_2$) ~3.7 (2H, m, H-23a,b) 4.24 (1H, d, H-1') 4.75 (1H, dd, H-2') 5.80 (1H, d, H-13) 6.35 (1H, d, H-10) 7.28 (1H, d, H-11) 9.68 (1H, s, 20-CHO)

Example 5

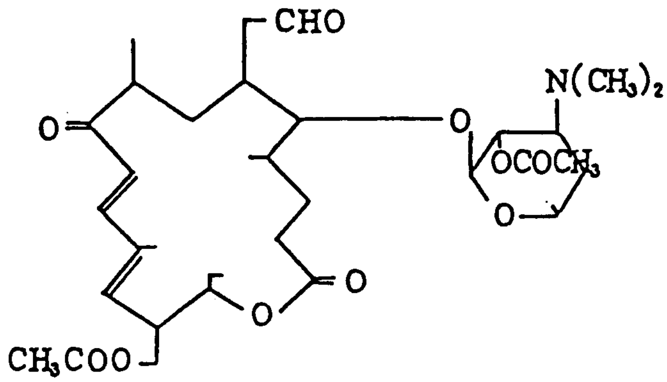

201 mg of 2′-O-acetyl-3,4′-dideoxy-mycaminosyl tylonolide was dissolved in 4 ml of dry pyridine, and after adding 47 µl of acetic anhydride, the mixture was kept at room temperature for 10 hours. The reaction solution was concentrated, and poured into 20 ml of saturated aqueous sodium hydrogen carbonate. The mixture was extracted with toluene, and the organic layer was washed with water, dried and concentrated to give pale yellow syrup. This was purified by silica gel column chromatography (Wako gel C-200: 10g; benzene:ethyl acetate = 1:3) to give colorless solid (211 mg, yield: 98%) 2′,23-di-O-acetyl-3,4′-dideoxy-mycaminosyl tylonolide.

Physico-chemical properties:

(i) $[\alpha]_D^{21}$ : -6° (c1, $CHCl_3$)
(ii)

| Elemental analysis ($C_{35}H_{55}NO_{10}$) | | | |
|---|---|---|---|
| | C | H | N |
| Found(%) | 64.64 | 8.55 | 1.96 |
| Calcd.(%) | 64.69 | 8.53 | 2.16 |

(iii) Mass m/z = 649($M^+$)
(iv) $^1$H-nmr ($CDCl_3$, TMS internal standard)
δ 2.06, 2.08 (each 3H, s, $CH_3$ of 2′-$OCOCH_3$, $CH_3$ of 23-O-$COCH_3$) 2.26 (6H, s, 3′-$N(CH_3)_2$) ~4.1 (2H, m, H-23a,b) 4.24 (1H, d, H-1′) 4.75 (1H, dd, H-2′) 5.78 (1H, d, H-13) 6.35 (1H, d, H-10) 7.27 (1H, d, H-11) 9.68 (1H, s, 20-CHO)

Example 6

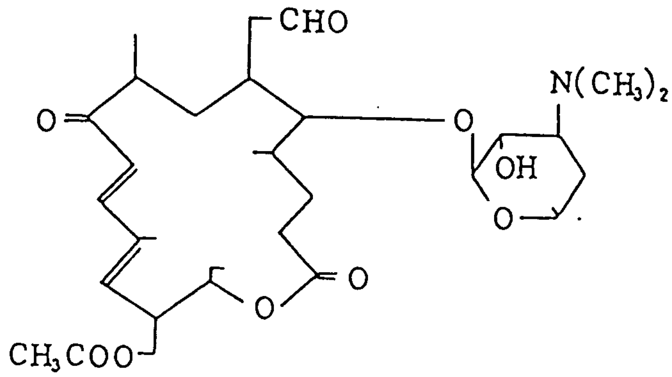

800 mg of 2′,23-di-O-acetyl-3,4′-dideoxymycaminosyltylonolide was dissolved in 16 ml of methanol, and the mixture was allowed to stand for 5 hours at 50 °C. The reaction solution was concentrated, and the residue was extracted with chloroform, washed with saturated aqueous sodium hydrogen carbonate and saturated aqueous sodium chloride successively, dried and concentrated to give pale yellow solid material. This was purified by silica gel column chromatography (Wako gel C-200: 30 g; chloroform:methanol = 10:1) to give 660 mg of colorless solid 23-O-acetyl-3,4′-dideoxy-mycaminosyltylonolide (yield: 88 %).

Physico-chemical character:

(i) $[\alpha]_D^{21}$ : -18° (c1, $CHCl_3$)
(ii)

| Elemental analysis ($C_{33}H_{53}NO_9$) | | | |
|---|---|---|---|
| | C | H | N |
| Found(%) | 65.26 | 8.93 | 2.10 |
| Calcd.(%) | 65.21 | 8.79 | 2.30 |

(ii) Mass m/z = 608 ($M^+$ + 1)
(iv) $^1$H-nmr ($CDCl_3$, TMS internal standard)
δ 2.06 (3H, s, $CH_3$ of 23-$OCOCH_3$) 2.26 (6H, s, 3′-$N(CH_3)_2$) ~4.2 (3H, m, H-1′, H-23a,b) 5.79 (1H, d, H-13) 6.36 (1H, d, H-10) 7.30 (1H, d, H-11) 9.70 (1H, s, 20-CHO)

Example 7

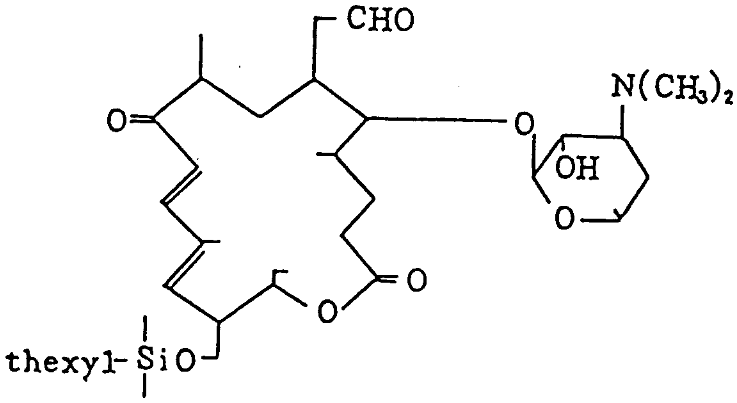

72 mg of 2'-O-acetyl-3,4'-dideoxy-mycaminosyl-tylonolide was dissolved in 1.4 ml of acetonitrile, and after adding 14.5 mg of imidazole and 28 µl of thexyldimethylsilane chloride, the mixture was allowed to stand at room temperature overnight The reaction solution was concentrated somewhat. After adding 10 ml of saturated aqueous sodium hydrogen carbonate, the mixture was extracted with ethyl acetate. The organic layer was washed with water , dried and concentrated to give 93 mg of pale yellow syrup. This was dissolved in 2.0 ml of methanol, and the mixture was allowed to react at 50 °C for 5 hours. The reaction solution was concentrated, and the residue was extracted with chloroform. The extract solution was washed with saturated aqueous sodium hydrogen carbonate and saturated aqueous sodium chloride, successively, dried and concentrated to give pale yellow solid material. This was purified by silica gel column chromatography (Wako gel C-200: 8 g; chloroform: methanol:conc. aqueous ammonia = 17:1:0.1) to give 69 mg (yield: 85 %) of 3,4'-di-deoxy-23-O-thexyldimethylsilyl-mycaminosyltylonolide (colorless solid material).

Physico-chemical character:

(i) $[\alpha]_D^{21}$ : -20 ° (c1, $CHCl_3$)
(ii)

| Elemental analysis ($C_{39}H_{69}NO_8Si$ 1/2$H_2O$) | | | |
|---|---|---|---|
| | C | H | N |
| Found(%) | 65.50 | 9.68 | 1.80 |
| Calcd.(%) | 65.32 | 9.70 | 1.96 |

(iii) Mass m/z = 708 ($M^+$)
(iv) $^1$H-nmr ($CDCl_3$, TMS)
δ 0.08x2 ( 3H, s, $-Si(CH_3)_2$ thexyl $CH_3$) 2.26 (6H, s, 3'-$N(CH_3)_2$) ~3.7 (2H, m, H-23a,b) 4.19 (1H, d, H-1') 5.88 (1H, d, H-13) 6.32 (1H, d, H-10) 7.30 (1H, d, H-11) 9.70 (1H, s, 20-CHO)

## Claims

**Claims for the following Contracting States : AT, BE, CH, LI, GB, DE, FR, IT, LU, NL, SE**

1. A 3-deoxy-mycaminosyltylonolide compound of formula (I) or a salt thereof

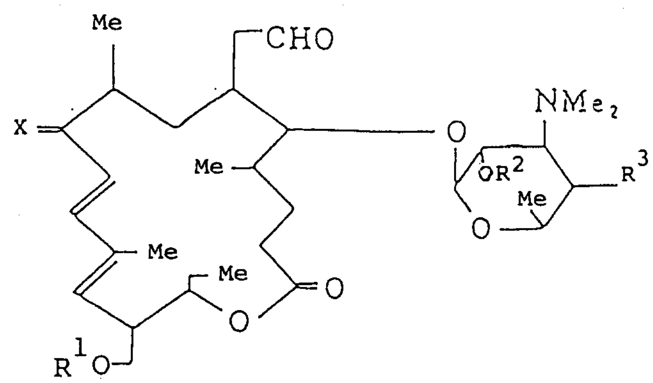

(I)

wherein X is O or =N-OR$^4$ (wherein $R^4$ is H or a $C_1$-$C_5$ alkyl group); $R^1$ is H or an acyl or alkylsilyl group; $R^2$ is H or an acyl group; and $R^3$ is H or a hydroxyl group.

2. A compound according to claim 1 which is 3,4'-dideoxy-mycaminosyltylonolide, 3-deoxy-mycaminosyltylonide, 9-deoxo-3,4'-dideoxy-9-N-methoxyiminomycaminosyltylonolide, 2'-O-acetyl-3,4'-dideoxy-mycaminosyltylonolide, 2',23-di-O-acetyl-3,4'-dideoxy-mycaminosyltylonolide, 23-O-acetyl-3,4'-dideoxy-mycaminosyltylonolide, or 3,4'-dideoxy-23-O-(2,3-dimethyl-2-butyl)dimethylsilylmycaminosyltylonolide

3. A pharmaceutical composition comprising compound according to claim 1 or 2 and pharmaceutical carrier or excipient.

4. The use of a compound according to claim 1 or 2 for the preparation of an antibacterial medicament.

5. An intermediate compound for the preparation of a compound according to claim 1, the intermediate compound being
23-O-thexyldimethylsilyl-mycaminosyl tylonolide 9,20-bis[ethyleneacetal),
2',4'-di-O-acetyl-23-O-thexyldimethylsilyl mycaminosyl tylonolide-9,20-bis[ethyleneacetal],
2',4'-di-O-acetyl-3-O-mesyl-23-O-thexyldimethylsilyl mycaminosyl tylonolide-9,20-bis-[ethyleneacetal],
2-dehydro-2-en-3-deoxy-23-O-thexyldimethylsilylmycaminosyl tylonolide 9,20-bis[ethyleneacetal],
4'-O-benzylsulfonyl-3-deoxy-23-O-thexyldimethylsilylmycaminosyl tylonolide 9,20-bis-[ethyleneacetal],
3,4'-dideoxy-4'-iodo-23-O-thexyldimethylsilyl-mycaminosyl tylonolide 9,20-bis[ethyleneacetal), or
3,4'-dideoxymycaminosyl tylonolide dimethylacetal, wherein "thexyl" is 2,3 dimethyl-2-butyl.

6. A method of preparing a compound according to claim 1 wherein X is O and $R^1$ and $R^2$ are H, which comprises reducing a compound of formula (II)

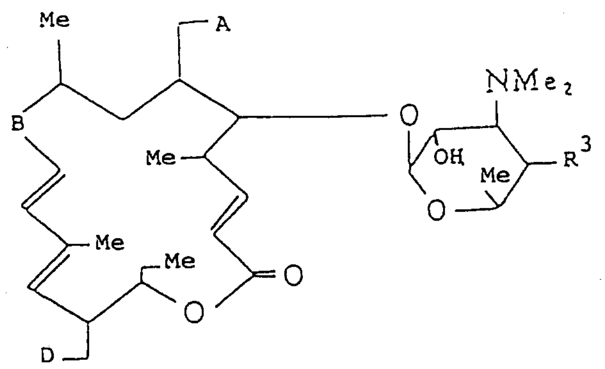

(II)

wherein $R^3$ is H or hydroxyl, A is protected or unprotected aldehyde, B is protected or unprotected carbonyl, and D is protected or unprotected hydroxy, and removing protective group(s) if desired.

7. A method of preparing a compound according to claim 1 wherein X is $R^4O\text{-}N=$, and $R^1$ and $R^2$ are H, which comprises reacting a compound of formula

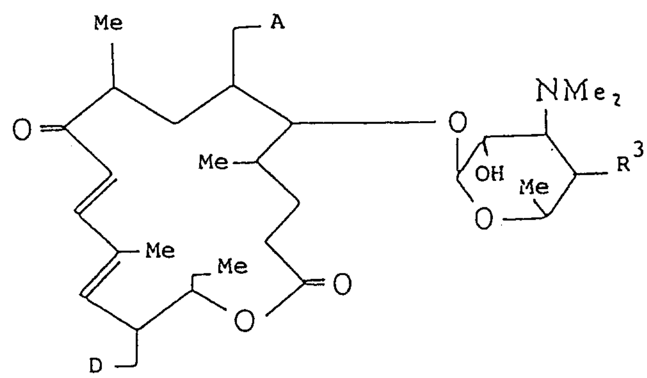

wherein $R^3$ is H or hydroxyl, A is protected or unprotected aldehyde and D is protected or unprotected hydroxyl, with compound of formula $H_2N\text{-}OR^4$ wherein $R^4$ is H or a $C_1\text{-}C_5$alkyl group, and removing protective group(s) if desired.

8. A method of preparing a compound according to claim 1 wherein X is O, and $R^1$, $R^2$ and $R^3$ are H, which comprises reacting a compound of formula (V)

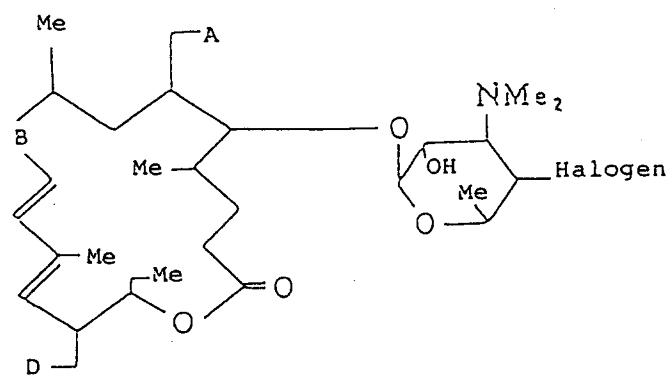

(V)

where A is protected or unprotected aldehyde, B is protected or unprotected hydroxyl, and D is protected or unprotected hydroxy, with trisubstituted tin hydride to replace halogen by hydrogen, and removing protective group(s) if desired.

9. A method of preparing a compound according to claim 1 wherein X is O, at least one of $R^1$ and $R^2$ is acyl and $R^3$ is H which comprises acylating the compound of formula (VI)

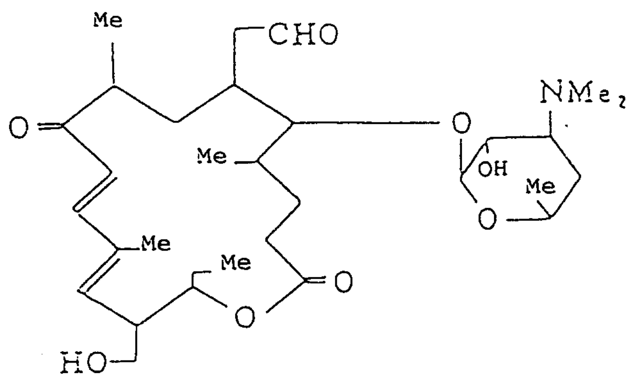

(VI)

10. A method of preparing a compound according to claim 1 wherein X is O, $R^1$ is alkylsilyl, $R^2$ is H or acyl and $R^3$ is H, which comprises reacting compound of formula (VII)

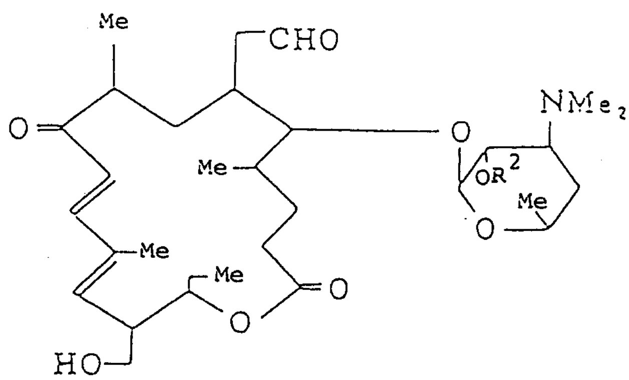

(VII)

with alkylsilyl halogen, and removing the 2'-acyl group if desired.

**Claims for the following Contracting States : ES, GR**

1. A process for forming a pharmaceutical composition which comprises combining pharmaceutical carrier or excipient with pharmaceutical compound selected from compounds of formula (I) and salts thereof

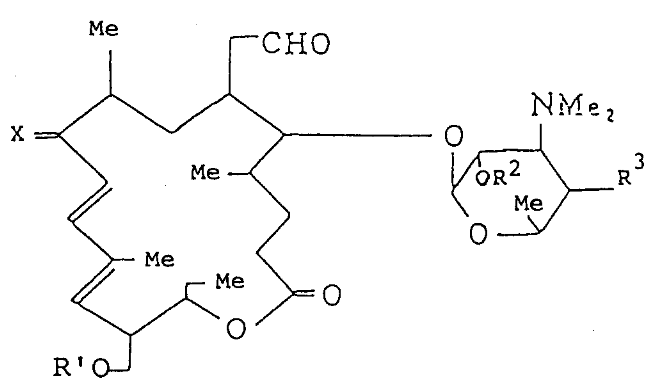

(I)

wherein X is O or =N-$OR^4$ (wherein $R^4$ is H or a $C_1$-$C_5$ alkyl group); $R^1$ is H or an acyl or alkylsilyl group; $R^2$ is H or an acyl group; and $R^3$ is H or a hydroxyl group.

2. The use of pharmaceutical compound selected from compounds of formula (I) and salts thereof for the preparation of an antibacterial medicament, said formula (I) being as defined in claim 1.

3. A method of preparing a pharmaceutical compound as defined in claim 1 wherein X is O and $R^1$ and $R^2$ are H, which comprises reducing a compound of formula (II)

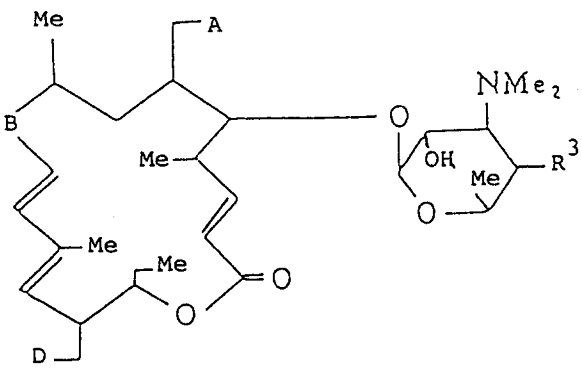

(II)

wherein $R^3$ is H or hydroxyl, A is protected or unprotected aldehyde, B is protected or unprotected carbonyl, and D is protected or unprotected hydroxy, and removing protective group(s) if desired.

4. A method of preparing a pharmaceutical compound as defined in claim 1 wherein X is $R^4O\text{-}N=$, and $R^1$ and $R^2$ are H, which comprises reacting a compound of formula

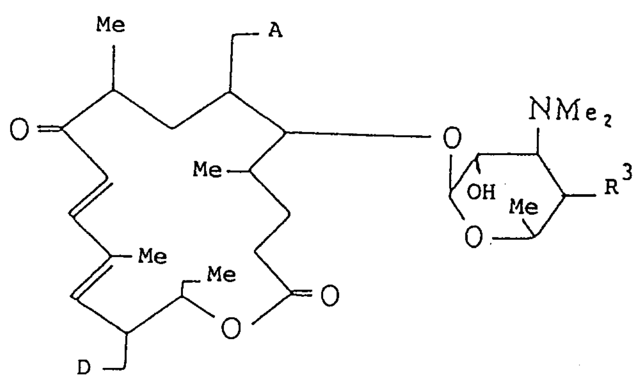

wherein $R^3$ is H or hydroxyl, A is protected or unprotected aldehyde and D is protected or unprotected hydroxyl, with compound of formula $H_2N\text{-}OR^4$ wherein $R^4$ is H or a $C_1$-$C_5$alkyl group, and removing protective group(s) if desired.

5. A method of preparing a pharmaceutical compound as defined in claim 1 wherein X is O, and $R^1$, $R^2$ and $R^3$ are H, which comprises reacting a compound of formula (V)

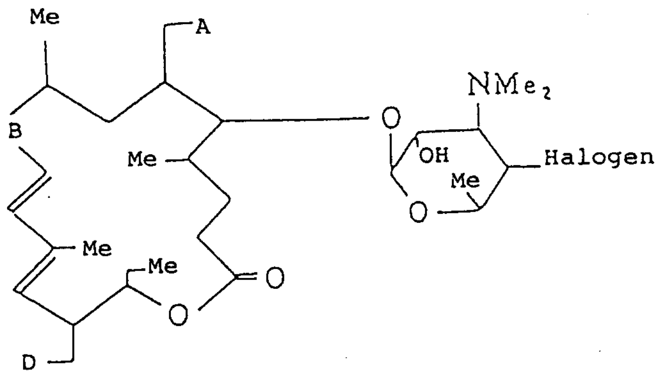

(V)

where A is protected or unprotected aldehyde, B is protected or unprotected hydroxyl, and D is protected or unprotected hydroxy, with trisubstituted tin hydride to replace halogen by hydrogen, and removing protective group(s) if desired.

**6.** A method of preparing a pharmaceutical compound as defined in claim 1 wherein X is O, at least one of $R^1$ and $R^2$ is acyl and $R^3$ is H which comprises acylating the compound of formula (VI)

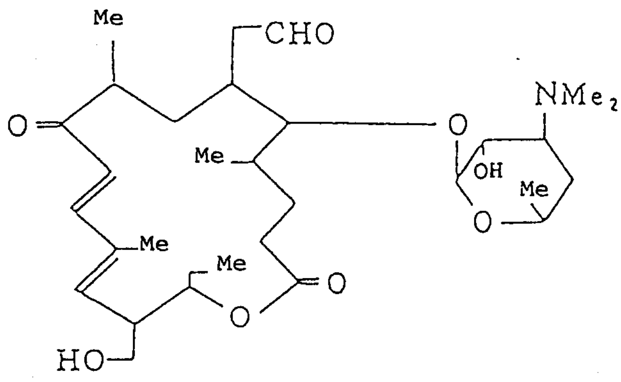

(VI)

**7.** A method of preparing a pharmaceutical compound as defined in claim 1 wherein X is O, $R^1$ is alkylsilyl, $R^2$ is H or acyl and $R^3$ is H, which comprises reacting compound of formula (VII)

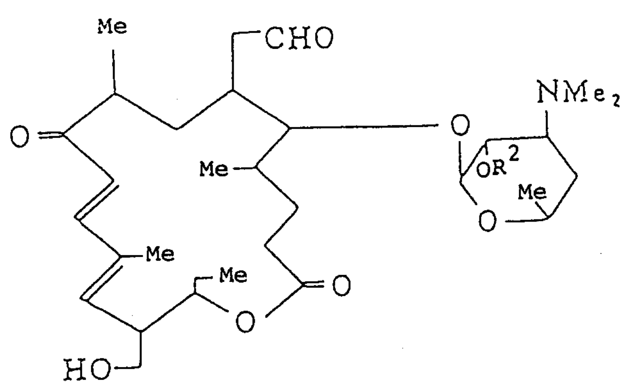

(VII)

with alkylsilyl halogen, and removing the 2'-acyl group if desired.

**8.** A process according to claim 1 wherein in formula (I) X is O and $R^1$, $R^2$ and $R^3$ are H.

**9.** A use according to claim 2 wherein in formula (I) X is O and $R^1$, $R^2$ and $R^3$ are H.

**Patentansprüche**
**Patentansprüche für folgende Vertragsstaaten : AT, BE, CH, LI, GB, DE, FR, IT, LU, NL, SE**

**1.** 3-Deoxymycaminosyltylonolidverbindung der Formel (I) oder ein Salz davon

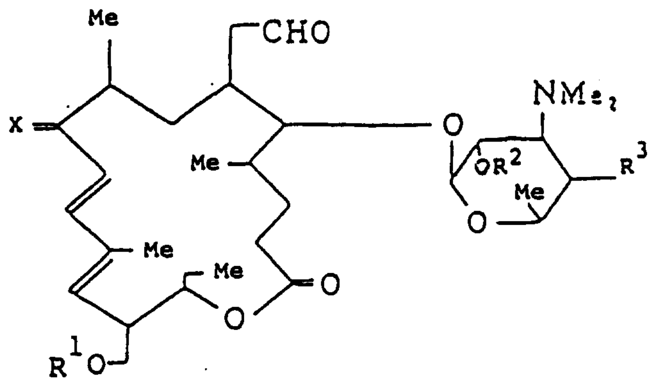

(I)

worin X die Bedeutung O oder $=N\text{-}OR^4$ hat (worin $R^4$ H oder eine $C_1$-$C_5$-Alkylgruppe ist); $R^1$ H oder eine Acyl- oder Alkylsilylgruppe ist; $R^2$ H oder eine Acylgruppe ist und $R^3$ H oder eine Hydroxylgruppe ist.

**2.** Verbindung nach Anspruch 1, welche 3,4'-Dideoxymycaminosyltylonolid, 3-Deoxmycaminosyltylonid, 9-Deoxo-3,4'-dideoxy-9-N-methoxyiminomycaminosyltylonolid, 2'-O-Acetyl-3,4'-dideoxymycaminosyltylonolid, 2',23-Di-O-acetyl-3,4'-dideoxymycaminosyltylonolid, 23-O-Acetyl-3,4'-dideoxymycaminosyltylonolid oder 3,4'-Didedoxy-23-O-(2,3-dimethyl-2-butyl)dimethylsilylmycaminosyltylonolid ist.

**3.** Pharmazeutische Zusammensetzung, welche eine Verbindung nach Anspruch 1 oder 2 und einen pharmazeutischen Träger oder Vehikel umfaßt.

**4.** Verwendung einer Verbindung nach Anspruch 1 oder 2 für die Herstellung eines antibakteriellen Medikaments.

**5.** Zwischenverbindung für die Herstellung einer Verbindung nach Anspruch 1, wobei die Zwischenverbindung 23-O-Thexyldimethylsilylmycaminosyltylonolid-9,20-bis[ethylenacetal],
2',4'-Di-O-acetyl-23-O-thexyldimethylsilylmycaminosyltylonolid-9,20-bis[ethylenacetal],
2',4'-Di-O-acetyl-3-O-mesyl-23-O-thexyldimethylsilylmycaminosyltylonolid-9,20-bis[ethylenacetal],
2-Dehydro-2-en-3-deoxy-23-O-thexyldimethylsilylmycaminosyltylonolid-9,20-bis[ethylenacetal],
4'-O-Benzylsulfonyl-3-deoxy-23-O-thexyldimethylsilylmycaminosyltylonolid-9,20-bis[ethylenacetal],
3,4'-Dideoxy-4'-iodo-23-O-thexyldimethylsilylmycaminosyltylonolid-9,20-bis[ethylenacetal] oder
3,4'-Dideoxymycaminosyltylonoliddimethylacetal ist, worin "thexyl" der 2,3-Dimethyl-2-butylrest ist.

**6.** Verfahren zur Herstellung einer Verbindung nach Anspruch 1, worin X die Bedeutung O und $R^1$ und $R^2$ die Bedeutung H haben, welches die Reduzierung einer Verbindung der Formel (II)

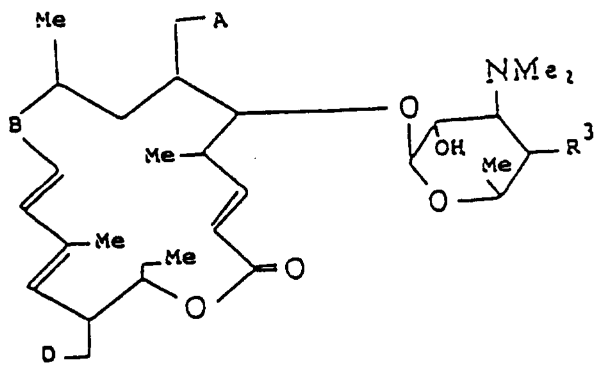

(II)

worin $R^3$ H oder Hydroxyl ist, A eine geschützte oder ungeschützte Aldehydgruppe ist, B eine geschützte oder ungeschützte Carbonylgruppe ist und D ein geschützter oder ungeschützter Hydroxyrest ist und gegebenenfalls die Entfernung der Schutzgruppe(n) umfaßt.

**7.** Verfahren zur Herstellung einer Verbindung nach Anspruch 1, worin X $R^4$O-N= ist und $R^1$ und $R^2$ H sind, welches die Umsetzung einer Verbindung der Formel

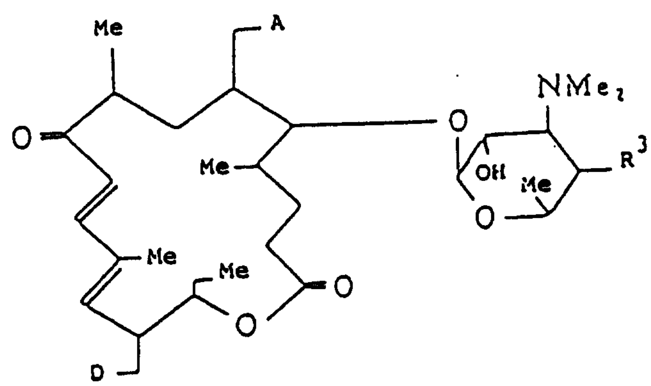

worin $R^3$ H oder Hydroxyl ist, A eine geschützte oder ungeschützte Aldehydgruppe ist und D ein geschützter oder ungeschützter Hydroxylrest ist, mit einer Verbindung der Formel $H_2N\text{-}OR^4$, worin $R^4$ H oder eine $C_1$-$C_5$-Alkylgruppe ist und gegebenenfalls die Entfernung der Schutzgruppe(n) umfaßt.

**8.** Verfahren zur Herstellung einer Verbindung nach Anspruch 1, worin X die Bedeutung O hat und $R^1$, $R^2$ und $R^3$ H sind, welches die Umsetzung einer Verbindung der Formel (V)

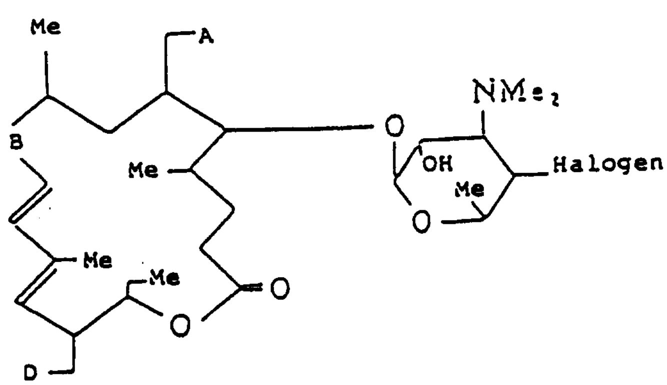

(V)

worin A eine geschützte oder ungeschützte Aldehydgruppe ist, B ein geschützter oder ungeschützter Hydroxylrest ist und D ein geschützter oder ungeschützter Hydroxyrest ist, mit trisubstituiertem

Zinnhydrid, um den Halogenrest durch Wasserstoff zu ersetzen und gegebenenfalls die Entfernung der Schutzgruppe(n) umfaßt.

**9.** Verfahren zur Herstellung einer Verbindung nach Anspruch 1, worin X die Bedeutung O hat, wenigstens einer der Reste $R^1$ und $R^2$ ein Acylrest ist und $R^3$ H ist, welches die Acylierung der Verbindung der Formel (VI) umfaßt:

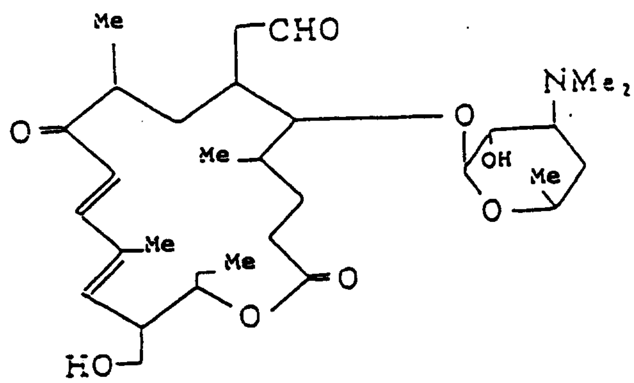

(VI)

**10.** Verfahren zur Herstellung einer Verbindung nach Anspruch 1, worin X die Bedeutung O hat, $R^1$ ein Alkylsilylrest ist, $R^2$ H oder Acylrest ist und $R^3$ H ist, welches die Umsetzung der Verbindung der Formel (VII)

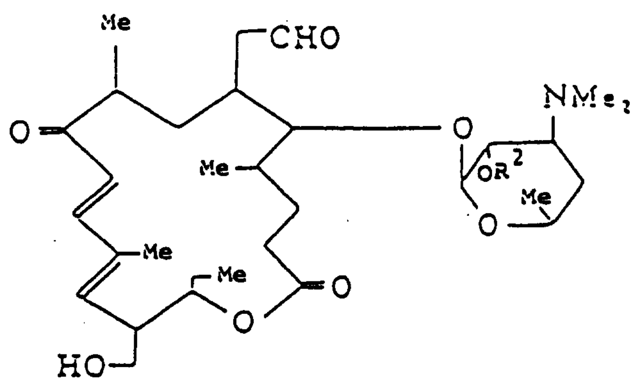

(VII)

mit Alkylsilylhalogen und gegebenenfalls die Entfernung der 2'-Acylgruppe umfaßt.

**Patentansprüche für folgende Vertragsstaaten : ES, GR**

**1.** Verfahren zur Bildung einer pharmazeutischen Zusammensetzung, welches das Verbinden eines pharmazeutischen Trägers oder Vehikels mit einer pharmazeutischen Verbindung, die aus Verbindungen der Formel (I) und Salzen davon ausgewählt ist, umfaßt

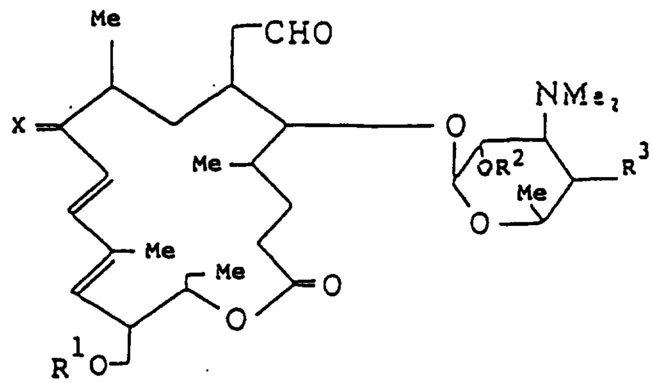

(I)

worin X die Bedeutung O oder $=N\text{-}OR^4$ hat (worin $R^4$ H oder eine $C_1$-$C_5$-Alkylgruppe ist); $R^1$ H oder eine Acyl- oder Alkylsilylgruppe ist; $R^2$ H oder eine Acylgruppe ist und $R^3$ H oder eine Hydroxylgruppe ist.

2. Verwendung der pharmazeutischen Verbindung, die aus Verbindungen der Formel (I) und Salzen davon ausgewählt ist, zur Herstellung eines anitbakteriellen Medikamentes, wobei die Formel (I) die in Anspruch 1 angegebene Bedeutung hat.

3. Verfahren zur Herstellung einer wie in Anspruch 1 definierten Verbindung, worin X die Bedeutung O hat und $R^1$ und $R^2$ die Bedeutung H haben, welches die Reduktion einer Verbindung der Formel (II)

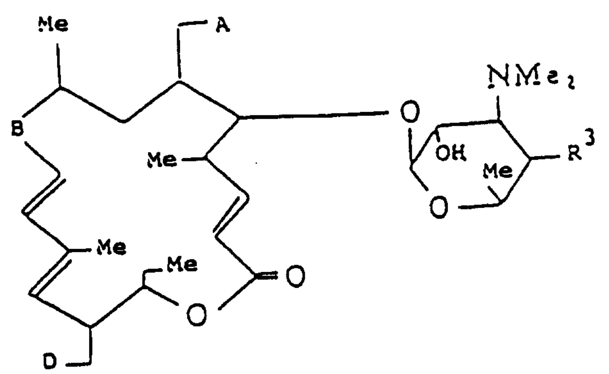

(II)

worin $R^3$ H oder Hydroxyl ist, A eine geschützte oder ungeschützte Aldehydgruppe ist, B eine geschützte oder ungeschützte Carbonylgruppe ist und D ein geschützter oder ungeschützter Hydroxyrest ist und gegebenenfalls die Entfernung der Schutzgruppe(n) umfaßt.

4. Verfahren zur Herstellung einer wie in Anspruch 1 definierten pharmazeutischen Verbindung, worin X $R^4O\text{-}N=$ ist und $R^1$ und $R^2$ H sind, welches die Umsetzung einer Verbindung der Formel

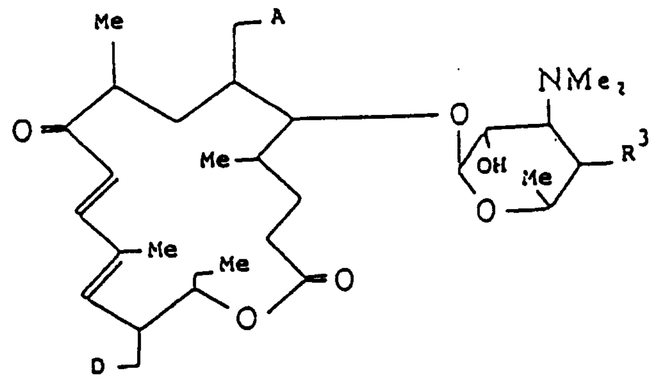

worin $R^3$ H oder Hydroxyl ist, A eine geschützte oder ungeschützte Aldehydgruppe ist und D ein geschützter oder ungeschützter Hydroxylrest ist, mit einer Verbindung der Formel $H_2N-OR^4$, worin $R^4$ H oder eine $C_1$-$C_5$-Alkylgruppe ist, und gegebenenfalls Entfernung der Schutzgruppe(n) umfaßt.

**5.** Verfahren zur Herstellung einer wie in Anspruch 1 definierten pharmazeutischen Verbindung, worin X die Bedeutung O hat und $R^1$, $R^2$ und $R^3$ H sind, welches die Umsetzung einer Verbindung der Formel (V)

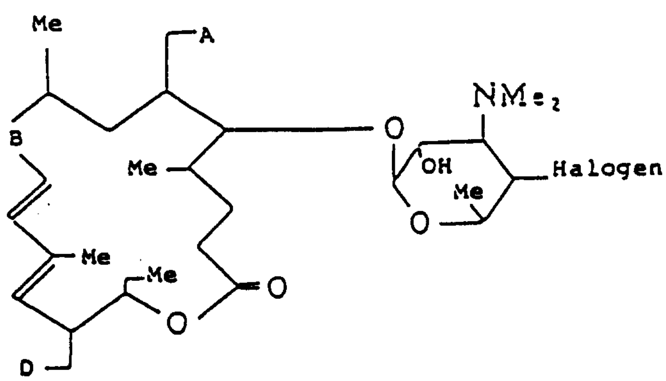

(V)

worin A eine geschützte oder ungeschützte Aldehydgruppe ist, B ein geschützter oder ungeschützter Hydroxylrest ist und D ein geschützter oder ungeschützter Hydroxyrest ist, mit trisubstituiertem Zinnhydrid, um Halogen durch Wasserstoff zu ersetzen, und gegebenenfalls die Entfernung der Schutzgruppe(n) umfaßt.

**6.** Verfahren zur Herstellung einer wie in Anspruch 1 definierten pharmazeutischen Verbindung, worin X die Bedeutung O hat, wenigstens einer der Reste $R^1$ und $R^2$ Acyl ist und $R^3$ H ist, welches die Acylierung der Verbindung der Formel (VI) umfaßt:

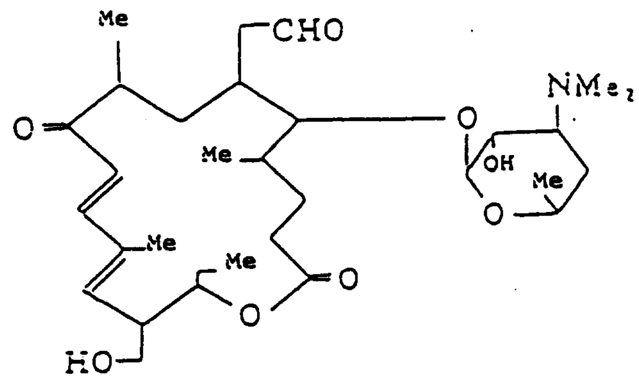

(VI)

7. Verfahren zur Herstellung einer wie in Anspruch 1 definierten Verbindung, worin X die Bedeutung O hat, $R^1$ ein Alkylsilylrest ist, $R^2$ H oder Acyl ist und $R^3$ H ist, welches die Umsetzung der Verbindung der Formel (VII)

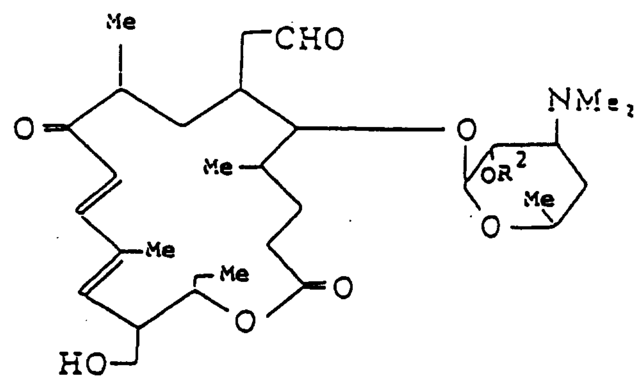

(VII)

mit Alkylsilylhalogen und gegebenenfalls die Entfernung der 2'-Acylgruppe umfaßt.

8. Verfahren nach Anspruch 1, worin in der Formel (I) X die Bedeutung O hat und $R^1$, $R^2$ und $R^3$ H sind.

9. Verwendung nach Anspruch 2, worin in der Formel (I) X die Bedeutung O hat und $R^1$, $R^2$ und $R^3$ H sind.

**Revendications**

**Revendications pour les Etats contractants suivants : AT, BE, CH, LI, GB, DE, FR, IT, LU, NL, SE**

1. Un composé 3-déoxy-mycaminosyltylonolide de formule (I) ou un sel de celui-ci

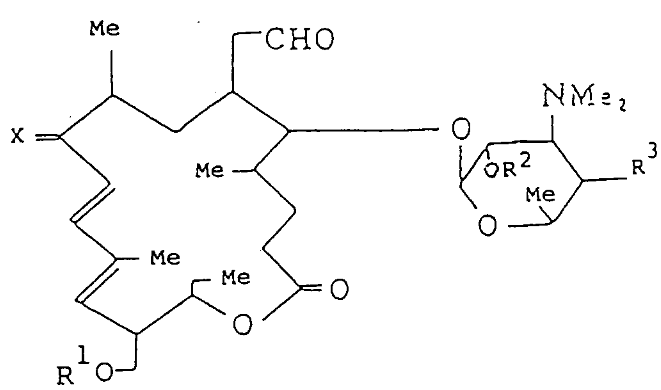

(I)

dans laquelle X est O ou =N-OR[4] (où $R^4$ est H ou un groupe alkyle en $C_1$-$C_5$) ; $R^1$ est H ou un groupe acyle ou alkylsilyle ; $R^2$ est H ou un groupe acyle ; et $R^3$ est H ou un groupe hydroxyle.

**2.** Un composé selon la revendication 1 qui est le 3,4'-didéoxy-mycaminosyltylonolide, le 3-déoxy-mycaminosyltylonide, le 9-déoxo-3,4'-didéoxy-9-N-méthoxyiminomycaminosyltylonolide, le 2'-0-acétyl-3,4'-didéoxy-mycaminosyltylonolide, le 2',23-di-O-acétyl-3,4'-didéoxy-mycaminosyltylonolide, le 23-0-acétyl-3,4'-didéoxy-mycaminosyltylonolide ou le 3,4'-didéoxy-23-0-(2,3-diméthyl-2-butyl)-diméthylsilylmycaminosyltylonolide.

**3.** Une composition pharmaceutique comprenant un composé conforme à la revendication 1 ou 2 et un support pharmaceutique ou un excipient.

**4.** L'utilisation d'un composé conforme à la revendication 1 ou 2 pour la préparation d'un médicament anti-bactérien.

**5.** Un composé intermédiaire pour la préparation d'un composé conforme à la revendication 1, le composé intermédiaire étant
le 23-0-thexyldiméthylsilyl-mycaminosyl tylonolide 9,20-bis[éthylèneacétal],
le 2',4'-di-0-acétyl-23-O-thexyldiméthylsilyl mycaminosyl tylonolide-9,20-bis[éthylèneacétal],
le 2',4'-di-0-acétyl-3-0-mésyl-23-0-thexyldiméthylsilyl mycaminosyl tylonolide-9,20-bis[éthylèneacétal],
le 2-déhydro-2-en-3-déoxy-23-0-thexyldiméthylsilylmycaminosyl
tylonolide 9,20-bis[éthylèneacétal],
le 4'-0-benzylsulfonyl-3-déoxy-23-0-thexyldiméthylsilylmycaminosyl tylonolide 9,20-bis[éthylèneacétal],
le 3,4'-didéoxy-4'-iodo-23-0-thexyldiméthylsilylmycaminosyl
tylonolide 9,20-bis[éthylèneacétal] ou
le 3,4'-didéoxymycaminosyl tylonolide diméthylacétal, dans lequel "thexyle" est le 2,3 diméthyl-2-butyle.

**6.** Un procédé de préparation d'un composé conforme à la revendication 1 dans lequel X est 0 et $R^1$ et $R^2$ sont H, qui comprend la réduction d'un composé de formule (II)

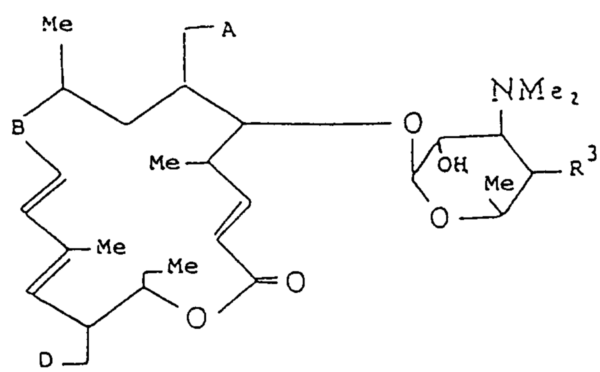

(II)

dans laquelle $R^3$ est H ou un hydroxyle, A est un aldéhyde protégé ou non protégé, B est un carbonyle protégé ou non protégé et D est un hydroxy protégé ou non protégé, et l'enlèvement du (des) groupe-(s) protecteur(s), si désiré.

**7.** Un procédé de préparation d'un composé conforme à la revendication 1 dans lequel X est $R^4$0-N= et $R^1$ et $R^2$ sont H, qui comprend la réaction d'un composé de formule

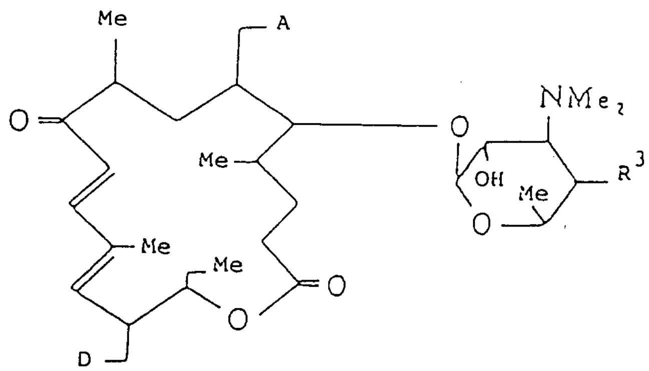

dans laquelle $R^3$ est H ou un hydroxyle, A est un aldéhyde protégé ou non protégé et D est un hydroxyle protégé ou non protégé, avec un composé de formule $H_2N\text{-}OR^4$ dans laquelle $R^4$ est H ou un groupe alkyle en $C_1$-$C_5$ et l'enlèvement du (des) groupe(s) protecteur(s), si désiré.

8. Un procédé de préparation d'un composé conforme à la revendication 1, dans lequel X est 0 et $R^1$, $R^2$ et $R^3$ sont H, qui comprend la réaction d'un composé de formule (V)

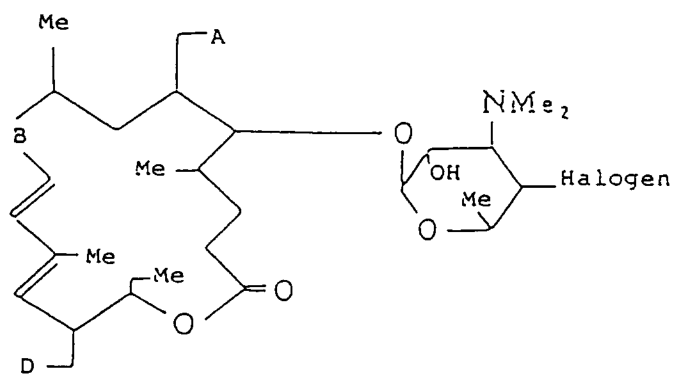

(V)

dans laquelle A est un aldéhyde protégé ou non protégé, B est un hydroxyle protégé ou non protégé et D est un hydroxy protégé ou non protégé, avec un hydrure d'étain trisubstitué pour remplacer l'halogène par de l'hydrogène et l'enlèvement du (des) groupe(s) protecteur(s), si désiré.

9. Un procédé de préparation d'un composé conforme à la revendication 1 dans lequel X est 0, au moins un de $R^1$ et $R^2$ est un acyle et $R^3$ est H, qui comprend l'acylation du composé de formule (VI)

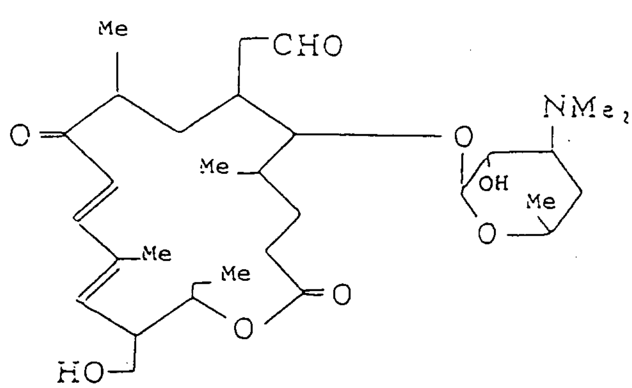

(VI)

10. Un procédé de préparation d'un composé conforme à la revendication 1 dans lequel X est 0, $R^1$ est un alkylsilyle, $R^2$ est H ou un acyle et $R^3$ est H, qui comprend la réaction d'un composé de formule (VII)

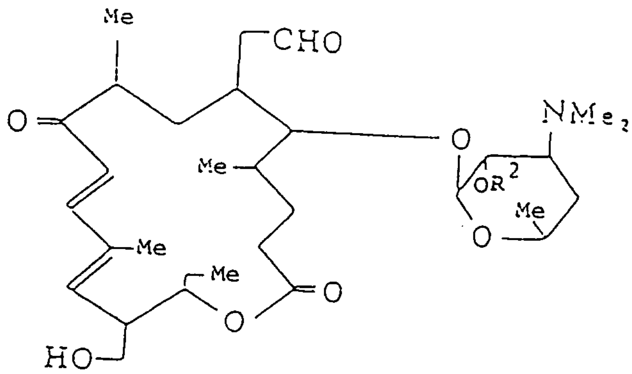

(VII)

avec un halogènure d'alkylsilyle, et l'enlèvement du groupe 2'-acyle, si désiré.

**Revendications pour les Etats contractants suivants : ES, GR**

**1.** Un procédé pour former une composition pharmaceutique qui comprend la combinaison d'un support ou d'un excipient pharmaceutique avec un composé pharmaceutique choisi parmi des composés de formule (I) et des sels de ceux-ci

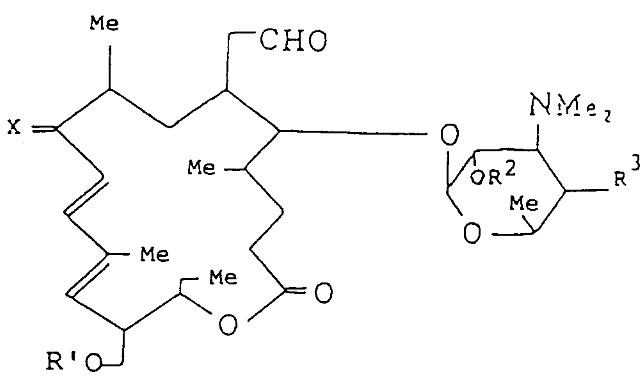

(I)

dans laquelle X est 0 ou =N-OR$^4$ (où R$^4$ est H ou un groupe alkyle en $C_1$-$C_5$) ; R$^1$ est H ou un groupe alkylsilyle ou acyle ; R$^2$ est H ou un groupe acyle ; et R$^3$ est H ou un groupe hydroxyle.

**2.** L'utilisation d'un composé pharmaceutique choisi parmi des composés de formule (I) et des sels de ceux-ci pour la préparation d'un médicament antibactérien, ladite formule (I) étant telle que définie dans la revendication 1.

**3.** Un procédé de préparation d'un composé pharmaceutique tel que défini dans la revendication 1, dans lequel X est 0 et R$^1$ et R$^2$ sont H, qui comprend la réduction d'un composé de formule (II)

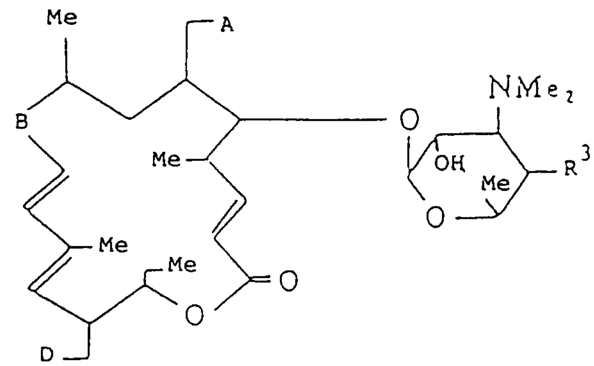

(II)

dans laquelle $R^3$ est H ou un hydroxyle, A est un aldéhyde protégé ou non protégé, B est un carbonyle protégé ou non protégé et D est un hydroxy protégé ou non protégé et l'enlèvement du (des) groupe(s) protecteur(s), si désiré.

4. Un procédé de préparation d'un composé pharmaceutique tel que défini dans la revendication 1 dans lequel X est $R^4$0-N=, et $R^1$ et $R^2$ sont H, qui comprend la réaction d'un composé de formule

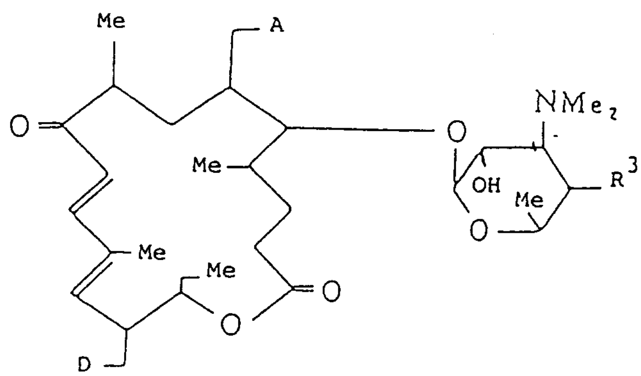

dans laquelle $R^3$ est H ou un hydroxyle, A est un aldéhyde protégé ou non protégé et D est un hydroxyle protégé ou non protégé, avec un composé de formule $H_2N\text{-}OR^4$ dans laquelle $R^4$ est H ou un groupe alkyle en $C_1$-$C_5$, et l'enlèvement du (des) groupe(s) protecteur(s), si désiré.

5. Un procédé de préparation d'un composé pharmaceutique tel que défini dans la revendication 1 dans lequel X est 0, et $R^1$, $R^2$ et $R^3$ sont H, qui comprend la réaction d'un composé de formule (V)

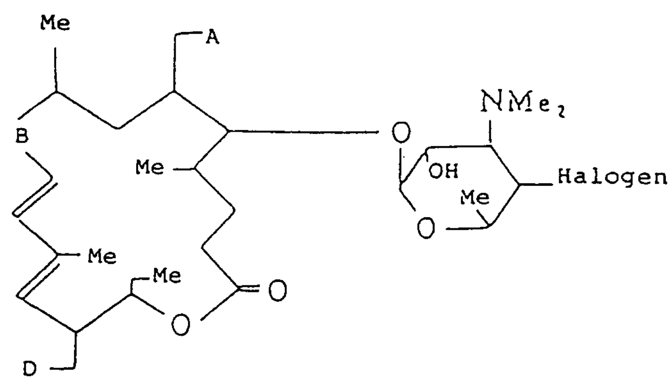

(V)

dans laquelle A est un aldéhyde protégé ou non protégé, B est un hydroxyle protégé ou non protégé et D est un hydroxy protégé ou non protégé, avec un hydrure d'étain trisubstitué pour remplacer l'halogène par de l'hydrogène, et l'enlèvement du (des) groupe(s) protecteur(s), si désiré.

6. Un procédé de préparation d'un composé pharmaceutique tel que défini à la revendication 1 dans lequel X est 0, au moins l'un de $R^1$ et $R^2$ est un acyle et $R^3$ est H, qui comprend l'acylation du composé de formule (VI)

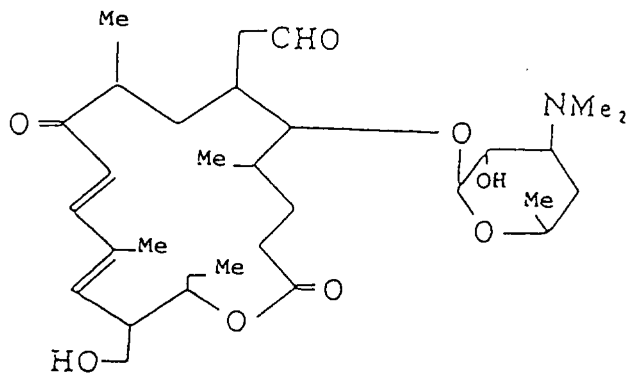

(VI)

7. Un procédé de préparation d'un composé pharmaceutique tel que défini dans la revendication 1 dans lequel X est 0, $R^1$ est un alkylsilyle, $R^2$ est H ou un acyle et $R^3$ est H, qui comprend la réaction d'un composé de formule (VII)

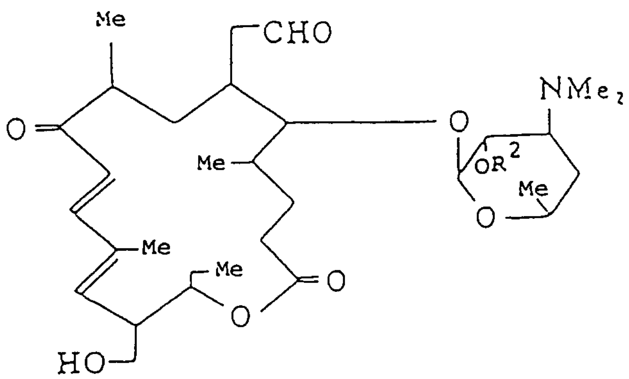

(VII)

avec un halogénure d'alkylsilyle, et l'enlèvement du groupe 2'-acyle, si désiré.

8. Un procédé conforme à la revendication 1 dans lequel, dans la formule (I), X est 0 et $R^1$, $R^2$ et $R^3$ sont H.

9. Une utilisation conforme à la revendication 2 dans laquelle, dans la formule (I), X est 0 et $R^1$, $R^2$ et $R^3$ sont H.